# EUROPÄISCHE PATENTSCHRIFT

(11) **EP 4 094 681 B1**
(45) Veröffentlichungstag und Bekanntmachung des Hinweises auf die Patenterteilung: **14.05.2025**
(21) Anmeldenummer: 21176722.3
(22) Anmeldetag: 28.05.2021
(51) Int. Cl.: A61B 5/0205, A61B 5/00, A61B 5/1455, A61B 5/08, A61B 5/024, A61B 5/11

(54) **GERÄT ZUR ERMITTLUNG EINES PHYSIOLOGISCHEN ZUSTANDS VON BABYS UND KLEINKINDERN**
DEVICE FOR DETERMINING A PHYSIOLOGICAL STATE OF BABIES AND INFANTS
DISPOSITIF DE DÉTERMINATION D'UN ÉTAT PHYSIOLOGIQUE DES NOURRISSONS ET DES PETITS ENFANTS

(43) Veröffentlichungstag der Anmeldung: 30.11.2022
(73) Patentinhaber: Lilio Health GmbH, 85748 Garching bei München (DE)
(72) Erfinder: WUNDERLICH, Nadine, 55469 Simmern (DE); WUNDERLICH, Björn, 55469 Simmern (DE)
(74) Vertreter: Richardt Patentanwälte PartG mbB

(56) Entgegenhaltungen:
- EP-A1- 3 480 824
- WO-A2-02/05702
- DE-A1- 102011 077 515
- DE-A1- 19 708 297
- US-A1- 2006 258 921
- US-A1- 2008 194 925
- US-A1- 2009 024 043
- US-A1- 2010 081 895
- US-A1- 2016 324 466
- US-A1- 2017 127 955
- US-A1- 2019 125 099
- US-A1- 2020 268 172
- US-A1- 2020 353 203
- US-B1- 6 498 652
- SHAMSIR SAMIRA ET AL: "Smart Infant-Monitoring System with Machine Learning Model to Detect Physiological Activities and Ambient Conditions", 2020 IEEE INTERNATIONAL INSTRUMENTATION AND MEASUREMENT TECHNOLOGY CONFERENCE (I2MTC), IEEE, 25 May 2020 (2020-05-25), pages 1 - 6, XP033785939, DOI: 10.1109/I2MTC43012.2020.9129295
- ABIRAMI E ET AL: "Design of infant monitoring system to reduce the risk of sudden infant death syndrome", 2017 INTERNATIONAL CONFERENCE ON COMMUNICATION AND SIGNAL PROCESSING (ICCSP), IEEE, 6 April 2017 (2017-04-06), pages 180 - 183, XP033315147, DOI: 10.1109/ICCSP.2017.8286802
- JOURAND P ET AL: "Robust monitoring of vital signs integrated in textile", SENSORS AND ACTUATORS A: PHYSICAL, ELSEVIER BV, NL, vol. 161, no. 1-2, 1 June 2010 (2010-06-01), pages 288 - 296, XP027120731, ISSN: 0924-4247, [retrieved on 20100521]
- FONSECA ANGELO M ET AL: "A sudden infant death prevention system for babies", 2014 IEEE 16TH INTERNATIONAL CONFERENCE ON E-HEALTH NETWORKING, APPLICATIONS AND SERVICES (HEALTHCOM), IEEE, 15 October 2014 (2014-10-15), pages 525 - 530, XP032715901, DOI: 10.1109/HEALTHCOM.2014.7001897
- KUMAR NAVIN ET AL: "Continuous vital sign analysis for predicting and preventing neonatal diseases in the twenty-first century: big data to the forefront", PEDIATRIC RESEARCH, LIPPINCOTT WILLIAMS & WILKINS, NEW YORK, US, vol. 87, no. 2, 4 August 2019 (2019-08-04), pages 210 - 220, XP036988463, ISSN: 0031-3998, [retrieved on 20190804], DOI: 10.1038/S41390-019-0527-0

## Beschreibung

### Gebiet

Die vorliegende Erfindung betrifft ein Gerät zur Bestimmung des physiologischen Zustands eines Babys oder Kleinkinds, welches am Körper des Kindes getragen wird und welche mehrere Sensoren enthält.

### Stand der Technik

Im Stand der Technik sind verschiedene Gerätschaften und Sensorsysteme zur Überwachung von Vitalparametern von Neugeborenen und Kleinkindern bekannt. In einem dezidiert medizinischen Kontext, z.B. auf einer Neugeborenenstation oder Intensivstation eines Krankenhauses zeichnen sich die Geräte oftmals durch hohe Präzision bei dem Erkennen insbesondere lebensbedrohlicher physiologischer Zustände aus. Allerdings sind die hierzu verwendeten Sensorsysteme oftmals sehr teuer, und es erfordert Zeit, Erfahrung und oftmals eine Vielzahl komplexer und großer Gerätschaften, die Sensoren korrekt am Körper des Kindes anzubringen. Diese für eine Neugeborenenstation konzipierten Geräte eignen sich somit nicht für die Überwachung physiologischer Zustände der Kinder in einem häuslichen Umfeld durch die Eltern des Kindes.

Im Häuslichen Umfeld gibt es mittlerweile einige Angebote für Eltern, zumindest einige physiologische Zustände ihrer Kinder mit vergleichsweise einfachen Mitteln überwachen zu können.

Die Patentanmeldung US2016324466A1 beschreibt ein Verfahren, Gerät und System zur lokalen und Überwachung von Umweltrisikofaktoren für den plötzlichen Kindstod (sudden infant death syndrome - SIDS). Das Gerät wird dazu verwendet, um die Schlafumgebung von Neugeborenen und Säuglingen zu Hause von einem Elternteil oder einer anderen Betreuungsperson zu überwachen. Das Gerät wird in der Nähe des Gesichts des Säuglings angebracht und überwacht z.B. den CO₂ Gehalt der ausgeatmeten Luft und die Liegeposition des Kindes. Insbesondere wird die Schlafposition und eine mögliche Bedeckung des Kopfes durch das Bettzeug als Risikofaktor angesehen, da diese die Atemwege blockieren und die Atmung beeinträchtigen können. Eine Überwachung von Blutparameterwerten erfolgt nicht.

Die Patentanmeldung US2018000405A1 offenbart ein System und Methoden zur Gesundheitsüberwachung. Das System erfasst verschiedene Vitalparameter der Mutter im Wochenbett sowie verschiedene Parameter des Neugeborenen wie z.B. fötale Herzrate und Oxygenierung. Es wird jedoch nicht beschrieben, dass Verfahren des maschinellen Lernens zur Vorhersage von physiologischen Parametern verwendet werden, insbesondere nicht zum Erkennen eines erhöhten Risikos für den plötzlichen Kindstod oder zum Erkennen eines Hungergefühls.

Die Patentanmeldung US 2020/0060590 A1 beschreibt einen Babymonitor bestehend aus einer Sensoreinheit und einer Empfangseinheit. Die Sensoreinheit enthält verschiedene Sensoren, eine Verarbeitungseinheit und eine Sendeeinheit. Die Verarbeitungseinheit verarbeitet die von den Sensoren gemessenen Rohdaten, insbesondere formatiert sie diese. Die Sendeeinheit sendet die formatierten Daten an die Empfangseinheit. Die Sensoreinheit wird am Fuß des Babys befestigt und enthält Sensoren zur Messung der Herzfrequenz, des Sauerstoffgehaltes des Blutes und der Bewegungsmessung. Die Herzfrequenz und der Sauerstoffgehalt werden mittels Pulsoximetrie gemessen. Die Empfangseinheit (nicht jedoch die Sensoreinheit) analysiert die empfangenen Daten und löst bei Bedarf einen Alarm aus.

Viele im Stand der Technik verwendeten Geräte zur Überwachung von Vitalparametern im häuslichen Umfeld weisen verschiedene Probleme auf. Oftmals enthalten sie nur wenige Sensoren, da eine größere Anzahl von Sensoren sich oftmals nicht einfach in Kleidungsstücke oder Accessoires integrieren lässt, die von Babys und Kleinkindern getragen werden können, da nur wenig Platz bzw. Fläche für die Anbringung der Sensoren zur Verfügung steht. Die geringe Anzahl an Sensoren bewirkt oftmals auch, dass die Datenbasis wenig umfangreich und darauf basierende Vorhersagen von schlechter Qualität sind. Die Aufnahme weiterer Sensoren würde das Gerät zudem oftmals deutlich verteuern.

Ein weiteres Problem einiger im Stand der Technik verwendeten Geräte ist, dass die Messdaten für sich alleine genommen für die Anwender oftmals nur von bedingtem Nutzen sind. Eine veränderte Atemfrequenz oder eine verringerte Sauerstoffkonzentration kann verschiedene Ursachen haben, sodass diese Werte alleine es den Eltern nicht erlauben, zu erkennen, ob ein Problem vorliegt.

Die US-Patentanmeldung US 2006/0258921 A1 beschreibt ein physiologisches Messsystem, das ein Pulsoximetriesignal, wie z.B. ein Photoplethysmogramm, von einem Patienten aufnehmen und dann das Signal analysieren kann, um physiologische Parameter, einschließlich Atmung, Puls, Sauerstoffsättigung und Bewegung, zu messen. Das System kann als allgemeines Überwachungssystem oder speziell für die Apnoe bei Säuglingen oder Erwachsenen und zum Schutz vor dem plötzlichen Kindstodsyndrom verwendet werden. Das System umfasst ein Pulsoximeter, das eine lichtemittierende Vorrichtung und einen Fotodetektor, der an einer Person angebracht werden kann, um ein Pulsoximetriesignal zu erhalten; eine Analog-Digital-Wandlereinrichtung, die so angeordnet ist, dass sie das Pulsoximetriesignal in ein digitales Pulsoximetriesignal umwandelt.

Die Patentanmeldung DE 10 2011 077 515 A1 beschreibt eine Vorrichtung zur elektronischen Körperüberwachung, insbesondere für Säuglinge. Die Vorrichtung umfasst ein Sensorteil und ein Anzeigeteil. Das Sensorteil weist eine Sensoreinrichtung zur Überwachung von Parametern eines Säuglings, eine Fixiereinrichtung zum Fixieren des Sensorteils an dem Säugling und eine erste Funkschnittstelle auf. Die Vorrichtung umfasst eine Auswerteeinrichtung zur Auswertung der Sensorsignale und zum Auslösen eines Alarms. DE 19708297 A offenbart einen SIDS (Sudden Infant Death Syndrome)-Monitor für Säuglinge. WO 02/05702 A2 offenbart einen nicht-invasiven Sensor zur Messung von Blutzucker und gibt einen Hungerindex aus, der auf Basis der Blutzuckerwerte bestimmt wird.

### Zusammenfassung

Der Erfindung liegt die Aufgabe zugrunde, ein verbessertes Gerät zur Erkennung physiologischer Zustände eines Babys oder Kleinkinds bereitzustellen, welches die oben genannten Probleme nicht oder in einem geringeren Maße aufweist.

Die der Erfindung zugrunde liegenden Aufgaben werden jeweils mit den Merkmalen der unabhängigen Patentansprüche gelöst. Ausführungsformen der Erfindung sind in den abhängigen Ansprüchen angegeben.

Die Erfindung umfasst ein Gerät nach Anspruch 1.

Dies kann vorteilhaft sein, da die obengenannten Parameter sich als besonders prädiktiv für eine Vielzahl von relevanten physiologischen Zuständen erwiesen haben, darunter insbesondere auch physiologische Zustände, bei welchen ein erhöhtes Risiko für den plötzlichen Kindstod besteht. Die Auswertung der besagten Parameter ist vorteilhaft, weil diese mittels Sensoren durchgeführt werden können, die unmittelbar am Körper angebracht werden können, sodass die erhaltenen Messwerte weniger anfällig sind von Relativbewegungen des Kindes zu externen Sensoren und weniger Anfällig sind bezüglich verschiedener externer Einflussfaktoren. Die Anmelderin hat beobachtet, dass die oben genannten drei Parameter hochprädiktiv für ein erhöhtes Risiko des plötzlichen Kindstodes sind. Zwar kann durch die Berücksichtigung weiterer Parameter die Genauigkeit weiter gesteigert werden, doch ist bereits auf der Basis der besagten drei Parameter eine hinreichend genaue Vorhersagequalität möglich, um einerseits zuverlässig vor Risikosituationen bezüglich SIDS zu warnen und andererseits nicht so viele Fehlalarme auszulösen, dass die Eltern sich veranlasst sehen würden, die Funktion zu deaktivieren. Externe Sensoren wie z.B. externe Kameras oder Mikrophone zur Überwachung der Lage oder Atmung des Kindes haben den Nachteil, dass das Kind sich aus dem Sensorbereich bewegen kann, sodass kritische Situationen ggf. nicht mehr erfasst werden. Außerdem ist von Nachteil, dass der Aufbau der Sensorumgebung so aufwändig ist, dass in vielen Situationen, z.B. bei Urlaubsreisen oder wenn das Kind auf dem Sofa des Wohnzimmers und nicht im Kinderbett liegt, die Sensorumgebung gar nicht zur Verfügung steht. Es entstehen dadurch Schutzlücken. Dadurch, dass das Gerät mit der entsprechenden Sensorik und Auswertungssoftware als "Wearable" ausgebildet ist, entfällt der Aufwand mit dem Aufbau der Sensorumgebung und es ist auch ausgeschlossen, dass das Kind sich von dem von den externen Sensoren überwachten Bereich entfernt.

Die erfassten besagten drei Mindest-Vitalparameter sind außerdem vergleichsweise wenig störanfällig: so muss eine erhöhte CO₂ Konzentration der Außenluft nicht unbedingt ein Hinweis auf Atemprobleme des Kindes sein. Es ist möglich, dass die Raumluft ganz allgemein verbraucht ist. Die Auswertung des Akustiksignals externer Mikrophone bezüglich der Atemgeräusche kann außerdem durch Hintergrundlärm wie z.B. Renovierungsarbeiten gestört sein oder dadurch, dass eine Decke vor das Mikrophon rutscht. Diese Probleme existieren bei den besagten drei Vitalparametern nicht.

In einem weiteren Vorteilhaften Aspekt ist es möglich, alle drei Parameter über den gleichen Sensor zu erfassen bzw. aus den Rohdaten eines einzigen Sensors abzuleiten, z.B. wenn ein photoplethysmograpischen Sensor, hier als PPG Sensor bezeichnet, verwendet wird.

Ausführungsformen der Erfindung können es ermöglichen, das Eintreten problematischer physiologischer Zustände schon vor deren tatsächlichen Eintreten vorherzusagen, sodass die Eltern bzw. Bezugspersonen rechtzeitig Gegenmaßnahmen ergreifen können.

Erfindungsgemäß umfasst das Gerät zumindest den oder die für die Erfassung bzw. Ableitung der besagten drei Vitalparameter benötigten Sensoren. Optional kann das tragbare Gerät eine Reihe weiterer Sensoren für weitere Vitalparameter und/oder ein oder mehrere Umgebungsparameter beinhalten. Das bedeutet, dass keine Verkabelung des Kindes erfolgen muss. Das Anlegen bzw. "Anziehen" des Geräts ist ausreichend, um die Vielzahl an Sensoren mit dem Körper des Kindes in Kontakt zu bringen. Somit ist das Kind in seinem natürlichen Bewegungsablauf nicht durch Kabel behindert und es ist sichergestellt, dass keine Schutzlücken dadurch entstehen, dass das Kind vorübergehend oder während einer Reise aus einer "überwachten" Umgebung genommen wird.

Anders als bei Systemen, die Vitalparameter des Kindes durch externe Sensoren messen, besteht hier auch nicht die Gefahr, dass die Messungen durch Relativbewegungen des Kindes zur externen Messeinheit verfälscht werden. Da das Gerät am Körper des Kindes befestig ist, macht es auch die Bewegungen des Kindes mit.

In einem weiteren vorteilhaften Aspekt wird der vorhergesagte physiologische Zustand des Kindes als Ergebnis der Vorhersage ausgegeben bzw. an das Telekommunikationsgerät übermittelt. Das Telekommunikationsgerät kann z.B. ein Smartphone der Eltern bzw. der Betreuungsperson sein. Der Nutzer muss also nicht einzelne physiologische Parameter interpretieren, sondern wird direkt über den wahrscheinlich vorliegenden physiologischen Zustand des Kindes informiert.

Zusätzlich oder alternativ dazu können einige der vom tragbaren Gerät erfassten oder abgeleiteten Daten, die Vorhersageergebnisse oder Zwischenergebnisse der Vorhersage auch an ein Server-Computersystem über ein Netzwerk übermittelt werden. Beispielsweise kann das Server-Computersystem die vom tragbaren Gerät empfangenen Daten weiterverarbeiten. Die Weiterverarbeitung kann z.B. darin bestehen, komplexere, rechnerisch aufwändige Analysen mit den Daten auszuführen und/oder die Rohdaten in einer Datenbank zu speichern. Die Weiterverarbeitung kann eine Kombination der Daten des tragbaren Geräts mit den Daten weiterer externer Sensoren beinhalten, um ein endgültiges Vorhersageergebnis bezüglich des zumindest einen physiologischen Zustands zu erhalten und dieses endgültige Vorhersageergebnis zu speichern und/oder an das Telekommunikationsgerät der Eltern über das Netzwerk zu senden.

In einem weiteren vorteilhaften Aspekt findet die Datenverarbeitung zumindest bezüglich derjenigen physiologischen Zustände, die eine sofortige Intervention durch die Betreuungspersonen erfordern, direkt auf dem Gerät statt.

Gemäß Ausführungsformen wird das Vorhersageergebnis, optional ergänzt um einige der von den Sensoren erfassten Parameterwerte (Rohdaten) an das Telekommunikationsgerät nur dann gesendet, wenn ein aktueller, kritischer physiologischer Zustand berechnet oder ein akut kritischer Vitalparameterwert oder Umgebungsparameterwert erfasst wurde oder wenn die Betreuungsperson über das Telekommunikationsgerät die Datenübertragung explizit (per Pull Funktion) angefordert hat.

Dies reduziert den Datenverkehr über das Netzwerk und kann auch die Lebenszeit der Batterie verlängern, denn die Vorbereitung der Daten zum Versand sowie der Versand selbst erfordert Rechenleistung und damit Energie. Auch der Betrieb des Funkmoduls insbesondere im "strahlungsnormalen" Betriebsmodus erfordert Energie.

### Vorhersage eines erhöhten Risikos für den plötzlichen Kindstod

Erfindungsgemäß ist der zumindest eine physiologische Zustand ein Zustand eines erhöhten Risikos des plötzlichen Kindstods. Die Auswertungssoftware ist dazu konfiguriert, zumindest die Herzfrequenz, die Sauerstoffsättigung, und die Atemfrequenz als Input zu verwenden, um das akute oder künftige Vorliegen eines erhöhten Risikos für den plötzlichen Kindstod vorherzusagen.

Beispielsweise kann die Auswertungssoftware ein prädiktives Modell zur Vorhersage des plötzlichen Kindstods beinhalten, hier als "SIDS-Modell" bezeichnet. Unter einem SIDS-Modell wird hier ein prädiktives Modell zur Vorhersage des erhöhten Risikos des plötzlichen Kindstods verstanden. Das SIDS-Modell ist dazu konfiguriert, zumindest die Herzfrequenz, die Sauerstoffsättigung, und die Atemfrequenz, sowie optional einige weitere Vital- und Umgebungsparameter als Input zu verwenden, um das Vorliegen eines erhöhten Risikos für den plötzlichen Kindstod vorherzusagen.

Bei dem SIDS-Modell handelt es sich vorzugsweise um ein auf maschinellem Lernen basierendem Modell, insbesondere einem neuronalen Netzwerk. Es sind jedoch auch alternative Ausführungsformen möglich wie z.B. ein regelbasiertes System.

Die Verwendung der besagten Parameter hat den Vorteil, dass ein erhöhtes Risiko für den plötzlichen Kindstod (SIDS) mit höherer Sensitivität und Spezifität erkannt werden kann, als dies bisher im Segment der Geräte für die Heimanwendung möglich war. Eine hohe Sensitivität ist hier besonders wichtig, da der plötzliche Kindstod zu den häufigsten Todesursachen von Babys und Kleinkindern gehört. Eine hohe Spezifität ist aber ebenfalls sehr wichtig, da jeder Fehlalarm zum einen für die Eltern sehr belastend ist, zum anderen eine zu hohe Fehlalarmquote auch das Risiko birgt, dass ein Alarm im Ernstfall ignoriert wird.

Die erhöhte Qualität der Vorhersage ist insbesondere auf die kombinierte Auswertung der besagten Parameter Herzfrequenz, Sauerstoffsättigung, und Atemfrequenz zurückzuführen.

Durch die kombinierte Auswertung der Herzfrequenz, Sauerstoffsättigung und Atemfrequenz kann ein erhöhtes Risiko für den plötzlichen Kindstod früher, bevor es zu einem Atemstillstand oder auffälligen Mustern in der Atmung kommt, erkannt werden. Eltern können dadurch früher alarmiert werden. Es wird wertvolle Zeit für die Verhinderung des plötzlichen Kindstods gewonnen.

Auffälligkeiten in der Atmung (Apnoen (Atemaussetzer), Unregelmäßigkeiten in der Atemfrequenz) deuten auf ein erhöhtes SIDS-Risiko hin noch bevor es zu einer Hypoxämie kommt. Im weiteren Verlauf der Pathophysiologie können Bradykardie (niedrigere Herzfrequenz) hinzukommen. Schließlich kommt es zu einem starken Abfall der Sauerstoffkonzentration im Blut (Hypoxämie) und das Kind schnappt nach Luft. Normalerweise würde das autonome Nervensystem die Sauerstoffunterfunktion erkennen und gegensteuern. Beim plötzlichen Kindstod kann diese Gegenreaktion jedoch aus bisher unbekannten Gründen ausbleiben. Als mögliche Ursache hierfür wird eine fehlende Reife des Autonomen Nervensystems vermutet. Im Folgenden kommt es zu einem weiteren Sauerstoffabfall und zum SIDS.

Gemäß Ausführungsformen der Erfindung ist die Auswertesoftware dazu ausgebildet, ein erhöhtes Risiko für das aktuelle oder künftige Eintreten von SIDS zu berechnen und abgestuft nach Dringlichkeit verschiedene (und z.B. nach Dringlichkeit in unterschiedlichen Farben kodierte) Alarmnachrichten zu erzeugen und direkt oder indirekt (über das Server-Computersystem) an das Telekommunikationsgerät der Betreuungsperson auszugeben:
- Bei unregelmäßigen Atemmustern (Apnoe <ZEITDAUER) und ansonsten normaler Herzfrequenz und Sauerstoffkonzentration: Warnung: Kinderarzt aufsuchen!
- Bei unregelmäßigen Atemmustern (Apnoe > ZEITDAUER) und ansonsten normaler Herzfrequenz und Sauerstoffkonzentration: Ausgabe eines akuten Alarms, Stufe 1, orange;
- Bei unregelmäßigen Atemmustern (Apnoe > ZEITDAUER) + Hypoxie + normale Herzfrequenz: Ausgabe eines akuten Alarms, Stufe 1, orange;
- Bei unregelmäßigen Atemmustern (Apnoe > ZEITDAUER) + Hypoxie + Bradykardie: Ausgabe eines akuten Alarms, Stufe 2, rot.

Bei dem Parameter ZEITDAUER handelt es sich vorzugsweise um einen Wert im Bereich von 12 bis 19 Sekunden, insbesondere einem Wert von 14 bis 17 Sekunden, z.B. 15 Sekunden, 16 Sekunden oder 16,5 Sekunden.

Durch die kombinierte Auswertung der Parameter ist eine frühere und zuverlässigere Warnung möglich. Kinder mit einem erhöhten Risiko für SIDS können frühzeitig erkannt werden, um den Betreuungspersonen frühzeitig eine entsprechende ärztliche Untersuchung zu empfehlen. Da vorzugsweise die erfassten Vitalparameter (Atemfrequenz, Herzfrequenz, Sauerstoffsättigung) in dem tragbaren Gerät, dem Telekommunikationsgerät der Betreuungspersonen oder dem Server-Computersystem gespeichert werden, können die Eltern dem Arzt dabei aussagekräftige Langzeitdaten bezüglich der Vitalparameter des Kindes zur Verfügung stellen, um diesem die Diagnose zu erleichtern. Außerdem können durch das tragbare Gerät exogene Stressoren wie z.B. thermischer Stress (durch Bauchlage oder zu warme Umgebungstemperatur), Obstruktion durch Bauchlage, Verdeck des Gesichts durch Bauchlage, Decken/Kissen) erkannt werden, sodass die Betreuungspersonen sofort eingreifen können.

Gemäß einer weiteren Ausführungsform beinhaltet das tragbare Gerät einen Temperatursensor zur Messung der Hauttemperatur des Kindes. Vorzugsweise beinhaltet das tragbare Gerät zudem einen Temperatursensor zur Messung der Umgebungstemperatur.

Die Körpertemperatur in Kombination mit der Umgebungstemperatur kann von der Auswertungssoftware dazu verwendet werden, die Exaktheit der Vorhersage des erhöhten SIDS-Risikos zu erhöhen. Die Kombination aus Körpertemperatur und Umgebungstemperatur erlaubt nämlich eine zumindest näherungsweise Ableitung der Körperkerntemperatur. Eine stark erhöhte Körperkerntemperatur kann z.B. auf einen Hitzestau hindeuten, welcher das SIDS-Risiko erhöhen kann. Durch die Auswertung der Hauttemperatur in Kombination mit der Umgebungstemperatur kann die Qualität der Vorhersage bezüglich des Vorliegens eines erhöhten SIDS-Risikos noch weiter gesteigert werden.

Gemäß mancher Ausführungsformen enthält das Gerät zudem einen Luftfeuchtigkeitssensor, dessen Messwerte bei der besagten Vorhersage ebenfalls berücksichtigt wird. Bei hoher Luftfeuchtigkeit kann das Kind Hitzestau noch schlechter durch erhöhte Transpiration kompensieren. Dadurch, dass diese Risikofaktoren (erhöhte Körpertemperatur, ggf. in Kombination mit der Umgebungstemperatur und optional zudem dem Feuchtigkeitsgrad der das Gerät umgebenden Luft) berücksichtigt werden, wird die Vorhersagequalität erhöht.

Gemäß manchen Ausführungsformen ist die Auswertungssoftware dazu konfiguriert, als Vorhersageergebnis nicht nur die Aussage zu erzeugen, ob ein erhöhtes SIDS-Risiko vorliegt, sondern auch die relevanten Risikoparameter selbst (also z.B. verringerte Sauerstoffkonzentration im Blut, veränderte Herz- oder Atemfrequenz, zu hohe Körper- oder Umgebungstemperatur etc.) auszugeben. Dadurch haben Eltern die Möglichkeit, den relevanten Risikofaktoren gezielt zu begegnen. Beispielsweise kann die Liegeposition des Kindes geändert, eine Decke entfernt oder die Zimmertemperatur durch Öffnen von Fenstern abgesenkt werden.

Vorzugsweise befindet sich das Thermometer zur Messung der lokalen Hauttemperatur des Kindes an der Tragestelle des Geräts, wobei das Thermometer vorzugsweise direkten Hautkontakt hat.

Gemäß Ausführungsformen besitzt das Gerät ein Thermometer zur Messung einer lokalen Hauttemperatur des Kindes sowie ein Thermometer zur Messung der Umgebungstemperatur.

Beispielsweise kann der Hauttemperatursensor an der Innenseite eines als Bändchens ausgebildeten Geräts angebracht sein, die in direktem Kontakt mit der Haut des Kindes steht. Der Umgebungstemperatursensor kann auf der Außenseite des Bändchens angebracht sein. Gemäß manchen Ausführungsformen kann der Außentemperatursensor jedoch auch als ein externer Sensor ausgebildet sein, der über eine Basisstation die Umgebungstemperaturdaten an das tragbare Gerät und/oder das Server-Computersystem sendet.

Dies kann vorteilhaft sein, da die Vorhersagequalität weiter erhöht wird. So ist eine an der Haut gemessene erhöhte Körpertemperatur weniger problematisch, wenn die Umgebungstemperatur hoch ist, da letztere auch die Hauttemperatur unmittelbar beeinflusst. Eine erhöhte Hauttemperatur bei niedrigen Außentemperaturen ist jedoch ein deutliches Zeichen, dass eine physiologische Überhitzung vorliegt, z.B. wegen zu vieler Decken auf dem Kind.

Gemäß Ausführungsformen leitet die Auswertungssoftware aus der gemessenen Hauttemperatur des Kindes die Kernkörpertemperatur oder Änderungen der Körperkerntemperatur ab. Die derart abgeleitete Körpertemperatur wird sodann zusammen mit der Umgebungstemperatur als Input an die Auswertungssoftware zur Vorhersage eines erhöhten SIDS-Risikos weitergegeben.

Verfahren zur Ableitung der Körperkerntemperatur aus der Hauttemperatur sind an sich bekannt, und sind z.B. für erwachsene Männer in folgender Publikation beschrieben: Eggenberger P, et al.: "Prediction of Core Body Temperature Based on Skin Temperature", Heat Flux, and Heart Rate Under Different Exercise and Clothing Conditions in the Heat in Young Adult Males. Front Physiol. 2018;9:1780. Published 2018 Dec 10. doi:10.3389/fphys.2018.01780. Auch für Kinder kann ein entsprechender Datensatz erzeugt werden, in welchem Hauttemperatur, Umgebungstemperatur und gleichzeitig unter verschiedenen Bedingungen gemessene Körperkerntemperatur miteinander verknüpft sind. Durch Ausführung z.B. einer Regressionsanalyse auf diesen Daten kann eine als Formel bzw. Gleichung spezifizierte Funktion oder ein auf maschinellem Lernen beruhendes prädiktives Modell erzeugt werden, welches in der Lage ist, die Körperkerntemperatur aus der Hauttemperatur abzuleiten. Die Anmelderin hat beobachtet, dass die Verwendung einer abgeleiteten Körpertemperatur anstatt der direkt gemessenen Hauttemperatur die Qualität der Vorhersage eines erhöhten Risikos des plötzlichen Kindstods noch weiter erhöhen kann, da die Körperkerntemperatur in geringerem Maße von Störungsparametern der Umgebung beeinflusst wird und stärker mit SIDS-Risiken korreliert als die Hauttemperatur.

Beispielsweise kann durch die Verwendung der abgeleiteten Körperkerntemperatur anstatt der Hauttemperatur der Effekt der Zentralisation des Blutes bei Fiebergeschehen sowie ein zeitliches Veränderungsmuster der Kernkörpertemperatur erkannt werden.

Gemäß Ausführungsformen ist die Auswertungssoftware zudem konfiguriert für das Erkennen des Vorliegens von und/oder für die Vorhersage des künftigen Vorliegens von Fieber. Die Auswertungssoftware kann ein Profil der Änderung der abgeleiteten Körperkerntemperatur und optional noch ein oder mehrere weitere Parameter als Input verwenden, um das aktuelle oder künftige Vorliegen von Fieber vorherzusagen. Falls Fieber vorhergesagt wird, wird eine entsprechende Nachricht (Fieberalarm) direkt von dem Gerät ausgegeben und/oder über die Schnittstelle an das Telekommunikationsgerät übertragen.

Somit kann durch die Analyse der Gesamtheit der Daten (z.B. Herzfrequenz, Atemfrequenz, Blutsauerstoffkonzentration, abgeleitete Körperkerntemperatur und optional auch Umgebungstemperatur) eine bessere Vorhersagequalität im Hinblick auf den plötzlichen Kindstod erzielt werden.

Nach Ausführungsformen umfassen die Sensoren einen photoplethysmograpischen Sensor, hier als PPG Sensor bezeichnet. Die Auswertungssoftware ist dazu ausgebildet, aus den vom PPG Sensor erfassten Signalen sowohl die Herzfrequenz als auch die Sauerstoffsättigung und die Atemfrequenz des Kindes abzuleiten und als Input der Auswertungssoftware zur Vorhersage eines aktuellen oder künftigen erhöhten Risikos des plötzlichen Kindstods zur Verfügung zu stellen.

Die Verwendung eines PPG-Sensors zur Ableitung der oben genannten Vitalparametern aus den PPG-Signalen kann aus mehreren Gründen vorteilhaft sein: zum einen wird dadurch Platz gespart, sodass in dem Gerät ohne weiteres zusätzliche Sensoren untergebracht werden können. Außerdem kann das Gerät günstiger produziert werden, es ist leichter und auch weniger störanfällig, da es eine geringere Anzahl an Sensoren besitzt als dies nötig wäre, wenn für jeden der besagten Parameter ein eigener Sensor installiert werden müsste. Die Anmelderin hat beobachtet, dass die von heutigen PPG-Sensoren erzeugten Daten genügend Informationen beinhalten, um die besagten Parameter ableiten zu können.

Beispielsweise kann der PPG-Sensor eine photoplethysmographische Sonde mit einem lichtemittierenden Element und einem lichtdetektierenden Element sein. Das lichtemittierende Element kann z.B. aus einem Laser oder einer Kombination mehrerer Laser bestehen. Das Spektrum und die Intensität des reflektierten Lichts der jeweiligen Laser gibt Auskunft über die Menge an Blut, die zu einem bestimmten Zeitpunkt durch das in der Nähe des PPG-Sensors befindliche Adersystem gepumpt wird, und lässt somit auch die Ableitung der Herzfrequenz aus den Rohdaten zu. Da das Ein -und Ausatmen einen Einfluss auf den arteriellen Blutfluss hat, ist die Ableitung der Atemfrequenz vom PPG Signal möglich. Auffälligkeiten der Atemfrequenz ist neben der Herzfrequenz und Sauerstoffsättigung ein wichtiger prognostischer Faktor für das SIDS Risiko.

Die vom PPG-erfassten Lichtsignale erlauben es, Schwankungen der pro Zeiteinheit transportierten Blutmenge zu erkennen. Da diese Schwankungen unter anderem durch den Herzschlag und die Atmung beeinflusst werden, kann die Auswertungssoftware aus den PPG-Sensordaten auch den Herzschlag und den Takt der Atmung erkennen.

Beispielsweise kann die Herzfrequenz wie folgt mit Hilfe eines PPG Sensors erfasst bzw. berechnet werden: der PPG Sensor beinhaltet ein oder mehrere Lichtquellen, z.B. LEDs bestimmter Wellenlängen, die Licht emittieren, die durch die Haut gehen und (unter anderem) auf Blutgefäße treffen. Das Licht wird vom Gewebe und den darin enthaltenen Gefäßen absorbiert, verstreut und reflektiert. Ein Photodetektor misst die Intensität des transmittierten oder reflektierten Lichts.

Da sich die Absorptionseigenschaften von Blut und anderen Gewebe-Komponenten unterscheiden, können Änderungen im Volumen der Blutgefäße im Plethysmogramm analysiert werden. Das wellenförmige Plethysmogramm setzt sich aus der "direct current" (DC-) und "alternating current" (AC-) Komponente zusammen. Die DC Komponente hängt hauptsächlich von der Struktur des Gewebes und dem mittleren Blutvolumen des arteriellen und venösen Bluts ab. Veränderungen in der Venenkapazität sind als Veränderungen in der DC-Komponente feststellbar. Die AC-Komponente spiegelt die Volumenänderung während der Systole und Diastole des Herzens wider. Auf Basis dieser Pulsatilität kann die Herzfrequenz bestimmt werden.

Vorzugsweise wird mittels des PPG Sensors nicht nur die Herzfrequenz, sondern auch auf die Atemfrequenz erfasst bzw. aus den Rohdaten abgeleitet, da die Atmung und das Herz-Kreislauf-System sich gegenseitig beeinflussen.

Die Inspiration und Exspiration führt durch Veränderungen im intrathorakalen Druck zu arteriellen und venösen Blutvolumenschwankungen. Durch intrathorakalen Unterdruck bei der Inspiration fällt der Druck in den Venen und der venöse Zufluss zum Herz wird erhöht. Vor allem der systolische Blutdruck fällt ab und die Herzfrequenz steigt an. Bei der Exspiration tritt der entgegengesetzte Effekt ein.

Durch diese respirationsabhängigen Schwankungen des Blutdrucks und der Herzfrequenz kommt es zu Schwankungen des Blutvolumens und somit zu Schwankungen der gemessenen Intensität am Photodetektor.

Somit kann der PPG Sensor dazu verwendet werden, auch die Atemfrequenz des Kindes zu ermitteln.

Die Ableitung der Atemfrequenz aus einem PPG Signal kann z.B. durchgeführt werden wie dies in Nilsson LM. Respiration signals from photoplethysmography. Anesth Analg. 2013 Oct;117(4):859-65. doi: 10.1213/ANE.0b013e31828098b2. Epub 2013 Feb 28. PMID: 23449854 beschrieben ist. Auf analoge Weise kann auch die Herzschlagfrequenz aus den PPG Daten abgeleitet werden.

Die Werte können durch sonstige Bewegungen des Kindes beeinflusst werden, Bewegung stellt also eine mögliche Fehlerquelle dar. Durch Verwendung von Filtern können jedoch Schwankungen der Durchblutung, die durch eine Bewegung des Kindes (außer der Atembewegung!) hervorgerufen werden, erkannt und herausgefiltert werden. Außerdem wird gemäß Ausführungsformen der Erfindung das PPG-Signals dazu verwendet, Blutparameter des Kindes zu ermitteln, insbesondere die Sauerstoffsättigung und vorzugsweise weitere Blutparameter, mittels welcher die Qualität/Exaktheit der Vorhersage eines erhöhten Risikos für den plötzlichen Kindstod verbessert und/oder mittels welcher andere physiologische Zustände vorhergesagt bzw. erkannt werden können.

Bei den weiteren Blutparametern, mittels welcher die Qualität/Exaktheit der Vorhersage eines erhöhten Risikos für den plötzlichen Kindstod verbessert werden kann (die also als "Kontroll-Blutparameter" dienen), handelt es sich insbesondere um Blutparameter, die mit der Blutsauerstoffkonzentration nicht korrelieren oder in negativer Weise korrelieren oder auf eine bekannte und nicht-lineare Weise positiv mit dem Blutsauerstoff korrelieren.

Ein Blutparameter ist ein Messwert, der sich aus einer bestimmten Eigenschaft des Blutes ergibt, z.B. der Konzentration eines bestimmten Moleküls im Blut.

Ein Blutparameter, der negativ mit der Blutsauerstoffkonzentration korreliert ist z.B. ein Blutparameter, der bei steigender Blutsauerstoffkonzentration an Stärke abnimmt und bei fallender Blutsauerstoffkonzentration an Stärke zunimmt, z.B. die CO₂-Konzentration im Blut.

Ein Blutparameter, der nicht mit der Blutsauerstoffkonzentration korreliert ist, also z.B. ein Blutparameter, dessen Stärke zumindest näherungsweise unabhängig ist von der Höhe der Blutsauerstoffkonzentration. Beispielsweise hängt die Konzentration von Carboxyhämoglobin im Wesentlichen ab von der Kohlenmonoxidkonzentration der Luft, nicht von der Sauerstoffkonzentration, da Kohlenmonoxid Sauerstoff vom Häm verdrängt.

Generell können jedoch auch sonstige Blutparameter als Kontroll-Blutparameter verwendet werden, die sich von einem Blutbestandteil ableiten, die in bekannter nicht-linearer Weise positiv mit der Sauerstoffkonzentration korrelieren, z.B. gemäß einer exponentiellen oder polynomialen Relation. Wenn beispielsweise ein Abfall der Sauerstoffkonzentration im Blut um 30% festgestellt wird, und ein eine bestimmte Blutkomponente, von dem bekannt ist, dass sie in Abhängigkeit von der Sauerstoffkonzentration z.B. dreifach so stark steigt oder fällt wie die Sauerstoffkonzentration, kann auch als Kontrollparameter verwendet werden: verringert sich nämlich die gemessene oder abgeleitete Konzentration dieser Blutkomponente ebenfalls genau wie die Sauerstoffkonzentration um 30%, so ist von einem Messfehler auszugehen, z.B. weil der PPG Sensor, von dessen Rohdaten sich sowohl die Sauerstoffkonzentration als auch der Kontrollparameter ableiten, verrutscht ist. Nimmt die besagte Blutkomponente bei einem Abfall der Sauerstoffkonzentration um 30% aber um 90% ab, so ist davon auszugehen, dass tatsächlich ein Abfall der Sauerstoffkonzentration im Blut vorliegt, da ein Fehler in der Sensorik z.B. wegen fehlenden Kontakts in den meisten Fällen linear und gleichwirkend in die Messungen aller Messwerte dieser Sensoren eingeht.

Gemäß manchen Ausführungsformen umfassen die einen oder mehreren Sensoren einen Sensor zur Erfassung zumindest eines Blutparameters des Kindes, wobei der Blutparameter z.B. eine CO₂ Konzentration im Blut, eine Methämoglobin-Konzentration und/oder eine Carboxyhämoglobin-Konzentration im Blut des Kindes ist.

Beispielsweise kann der Sensor zur Erfassung des Blutparameters der PPG-Sensor sein, der bereits zur Erfassung des Blutsauerstoffgehalts, der Atemfrequenz und des Herzschlags verwendet wird. Dies ist vorteilhaft, weil kein zusätzlicher Sensor erforderlich ist und der gleiche Sensor, der bereits die Sauerstoffkonzentration im Blut misst bzw. aus den Rohdaten ableitet, verwendet werden kann.

Die Auswertungssoftware ist dazu ausgebildet, den zumindest einen Blutparameter (CO₂ Konzentration, Methämoglobin-Konzentration und/oder eine Carboxyhämoglobin-Konzentration im Blut) als zusätzlichen Inputparameter zu verwenden, um die die Falsch-Positivrate der Vorhersage des erhöhten Risikos des plötzlichen Kindstods zu reduzieren.

Zusätzlich oder alternativ zu den besagten drei Mindest-Vitalparametern Herzschlag, Atemfrequenz und Blutsauerstoffkonzentration können also auch ein oder mehrere der Kontroll-Blutparameter CO₂-Konzentration, Methämoglobin und/oder Carboxyhämoglobin-Konzentration im Blut als Kontrollparameter bei der Vorhersage eines erhöhten SIDS-Risikos verwendet werden.

Carboxyhämoglobin (HbCO) entsteht durch reversible Bindung von Kohlenmonoxid (CO) an das Eisenion der Hämgruppe. Kohlenmonoxid bindet an Hämoglobin an den gleichen Stellen wie Sauerstoff, jedoch etwa 200-mal stärker. Dadurch kann HbCO nahezu keinen Sauerstoff mehr binden. Da in der normalen Raumluft Kohlenmonoxid nicht oder nicht in nennenswerten Mengen vorkommt, ist unter Normalbedingungen von einem konstant bleibenden Wert für die CO-Konzentration auszugehen. Wenn neben einer zu niedrigen Sauerstoffkonzentration auch eine zu niedrige Carboxyhämoglobin-Konzentration gemessen wird, ist davon auszugehen, dass ein Messfehler vorliegt. Falls der Carboxyhämoglobin-Wert dagegen konstant bleibt, kann die Auswertungssoftware davon ausgehen, dass die Sauerstoffkonzentration tatsächlich erniedrigt ist.

Methämoglobin ist eine Form des Hämoglobins, welche ebenfalls keinen Sauerstoff mehr transportieren kann. Sie entsteht durch Oxidation des zweiwertigen Eisens (Fe2+) in der Hämgruppe zu dreiwertigem Eisen (Fe3+). Die physiologische Konzentration an Methämoglobin im Blut ist mit unter 1 % gering, die Konzentration kann jedoch durch bestimmte chemische Verbindungen ansteigen.

Methämoglobin ist wie auch Carboxyhämoglobin ein Blutparameter, der im Blut im Allgemeinen in gleichbleibender Konzentration vorliegt und daher als Kontrollparameter verwendet werden kann. Der Methämoglobingehalt wird gemäß Ausführungsformen als weiterer Eingabeparameter ("Kontrollparameter") an die Auswertungssoftware übergeben, sodass dieses durch einen Vergleich der eingegebenen Blutsauerstoffkonzentration mit diesem oder anderen Kontrollwerten erkennen kann, ob ein Messfehler vorliegt oder die Sauerstoffkonzentration tatsächlich zu niedrig ist.

Die Messung der Konzentration der besagten Stoffe im Blut bzw. die Ableitung dieser Konzentration aus den PPG-Sensorsignalen kann vorteilhaft sein, weil die besagten Blutparameter jeweils als Kontrollparameter verwendet werden können, um falsch positive Vorhersagen und Fehlalarme zu vermeiden. Wird z.B. eine zu niedrige Sauerstoffkonzentration gemessen, kann die Auswertungssoftware anhand eines oder mehrerer der besagten Kontroll-Blutparameter erkennen, ob wirklich ein erhöhtes Risiko für den plötzlichen Kindstod vorliegt, oder ob ein Messfehler die Ursache für die zu niedrige Sauerstoffkonzentration ist. Derartige Messfehler können durch Bewegung des Geräts, wenn das Kind sich bewegt, auftreten. Ist die Sauerstoffkonzentration im Blut deutlich erniedrigt, gleichzeitig aber die CO₂ Konzentration im Blut im Normbereich oder gar erhöht, ist es wahrscheinlich, dass tatsächlich eine zu niedrige Sauerstoffkonzentration im Blut vorliegt. Ist auch die Konzentration des CO₂ (oder einer anderen Kontrollsubstanz wie z.B. Carboxyhämoglobin oder Methämoglobin) im Blut erniedrigt, ist es wahrscheinlich, dass eine Fehlmessung zu Grunde liegt. CO₂ als Kontrollsubstanz müsste im Falle einer realen Senkung der Sauerstoffsättigung ansteigen, im Falle eines Fehlalarms würde CO₂ ebenfalls wie O2 sinken.

Dadurch, dass die Auswertungssoftware zusätzliche Kontrollparameter als Eingabeparameter verwendet und berücksichtigt, können Fehlalarme vermieden werden, was im Kontext der Erkennung eines lebensbedrohlichen physiologischen Zustands von besonderer Bedeutung ist.

Nach Ausführungsformen umfasst das Gerät mindestens einen Sensor zur Ermittlung mindestens eines weiteren Vitalparameters und/oder Umgebungsparameters.

Zusätzlich oder alternativ zu dem Sensor für den Umgebungsparameter kann das Gerät auch eine Schnittstelle zum Empfang des bzw. der weiteren Vitalparameters und/oder Umgebungsparameters von einem oder mehreren externen Sensor beinhalten. Beispielsweise können Daten auch von einem weiteren Bauteil, das im Zimmer angebracht ist, in dem sich das Kind aufhält, ermittelt und an das Gerät am Körper des Kindes und/oder an das Server-Computersystem übertragen werden. Der zumindest eine weitere Umgebungsparameter kann insbesondere die CO₂-Konzentration der Umgebungsluft oder die Luftfeuchte sein. Der mindestens eine weitere Vitalparameter kann Videodaten oder Bewegungsdaten beinhalten, welche die Bewegungsaktivität des Kindes charakterisieren. Es können auch akustische Daten von einem Mikrophon, das in dem tragbaren Gerät verbaut oder als externer Sensor ausgebildet ist, an das tragbare Gerät und/oder das Server-Computersystem übertragen werden und als weitere Eingabedaten bei der Vorhersage eines erhöhten SIDS-Risikos verwendet werden.

Die Auswertungssoftware ist dazu ausgebildet, den zumindest einen weiteren Vitalparameter und/oder Umgebungsparameter als zusätzlichen Inputparameter zu verwenden, um das Vorliegen eines erhöhten Risikos des plötzlichen Kindstods vorherzusagen.

Beispielsweise können ein oder mehrere Sensoren in der Matratze eines Kinderbetts oder als Aufkleber auf dem Bettbezug, dem Schlafanzug oder dem Schlafsack angebracht sein. Bei den externen Sensoren kann es sich z.B. um Bewegungssensoren oder Drucksensoren handeln, die z.B. die Bewegung des Brustkorbs im Schlaf messen. Zusätzlich oder alternativ dazu kann ein Bewegungssensor, z.B. ein Gyroskop, auch im tragbaren Gerät verbaut sein. Gemäß Ausführungsformen werden auch diese Bewegungsdaten als weiterer Eingabeparameter von der Auswertungssoftware dazu verwendet, die Falsch-Positiv-Rate der Vorhersagen zu reduzieren und die Qualität der Vorhersage zu verbessern: bei einem Kind, dessen Brustkorb sich bewegt, kann dies ein Hinweis darauf sein, dass die Atmung normal funktioniert und eine verringerte Sauerstoffkonzentration im Blut wohl auf einem Messfehler beruht. Somit kann insbesondere das Mikrophon und/oder die Videokamera und deren Messdaten dazu verwendet werden, die falsch-positiv-Rate der SIDS-Vorhersage zu reduzieren.

Gemäß einer weiteren Ausführungsform misst ein externer oder Geräte-interner Sensor die CO₂-Konzentration der Umgebungsluft. Dieser Parameter kann als weiterer Eingabeparameter von der Auswertungssoftware verwendet werden, um die Vorhersagegenauigkeit zu erhöhen. Ein hoher CO₂ Gehalt der Umgebungsluft ist ein Hinweis darauf, dass die Umgebungsluft verbraucht ist. Ist der CO₂-Wert zu hoch, weist das auf ungünstige Umgebungsbedingungen hin, die das Risiko für den plötzlichen Kindstod erhöhen können.

Gemäß einer weiteren Ausführungsform erfasst ein externer oder Geräte-interner Sensor für akustische Signale (ein Mikrophon) Geräusche des Kindes und der Umgebung (da ein Mikrophon sowohl die Geräusche der Umgebung als auch die des Kindes erfasst, ist es sowohl ein Umgebungssensor als auch ein Vitalparametersensor). Das erfasste akustische Signal kann als weiterer Eingabeparameter verwendet werden, um die Vorhersagegenauigkeit zu erhöhen. Falls das Kind schreit, liegen möglicherweise andere Probleme vor, aber kein Sauerstoffmangel oder ein erhöhtes Risiko für den plötzlichen Kindstod.

Zusätzlich oder alternativ dazu können die Bewegungsdaten des Beschleunigungssensors des tragbaren Geräts und/oder Videodaten einer externen auf das Kind gerichteten Kamera dazu verwendet werden, um die Bewegung des Kindes oder Bewegungsmuster zu erfassen und diese Bewegungsdaten der Auswertungssoftware als Eingabedaten zur Verfügung zu stellen. Bei einem Kind, das sich stark bewegt, kann man davon ausgehen, dass kein erhöhtes Risiko für den plötzlichen Kindstod vorliegt.

Gemäß einer Ausführungsform handelt es sich bei der Videokamera um eine Infrarot-Kamera. Dies ist besonders vorheilhaft, da die Bilder einer IR-Kamera es einer Bildanalysesoftware ermöglichen, zu erkennen, ob das Gesicht eines Kindes, das sich in einer IR-Kamera in der Regel deutlich vor dem Hintergrund wärmeisolierender Kleidung oder Decken abzeichnet, nach oben oder unten zeigt und damit anzeigt, ob sich das Kind in Bauch- oder Rückenlage befindet. Eine Bauchlage erhöht das Risiko des plötzlichen Kindstods, da das Kind in die Matratze atmet und/oder weil der Temperaturaustausch eingeschränkt sein kann.

Gemäß einer Ausführungsform werden die Vorhersageergebnisse des tragbaren Geräts bezüglich des Vorliegens eines erhöhten Risikos für das Eintreten des plötzlichen Kindstods als Zwischenergebnis zunächst an das Server-Computersystem übermittelt. Das Server-Computersystem ist über ein Netzwerk operativ an die IR-Kamera gekoppelt, z.B. direkt oder indirekt über eine Basisstation. Das Server-Computersystem empfängt die IR-Bilder von dem Kind von der externen Kamera und wertet diese mit einer Bildanalysesoftware aus. Die Bildanalyse ist rechnerisch vergleichsweise aufwändig, sodass diese Analyse vorzugsweise auf dem Server und nicht auf dem tragbaren Gerät ausgeführt wird, welches nur über begrenzte Rechenleistung verfügt. Das Ergebnis der Bildanalyse ist, ob das Kind in Bauchlage oder Rückenlage ist. Das Server-Computersystem ist dazu ausgebildet, aus dem Zwischenergebnis des tragbaren Geräts und dem Ergebnis der Bildanalyse ein Endergebnis bezüglich des Vorliegens eines erhöhten Risikos des plötzlichen Kindstods zu berechnen und an das Telekommunikationsgerät der Betreuungspersonen zu senden.

### Vorhersage von Hungergefühlen

Erfindungsgemäß ist zumindest einer der Sensoren für die Vitalparameter dazu ausgebildet, die Blutzuckerkonzentration des Kindes in nicht-invasiver Weise zu bestimmen. Die Auswertungssoftware ist ausgebildet zur Erkennung eines aktuell vorliegenden oder künftigen Hungergefühls des Kindes. Die Auswertungssoftware ist dazu konfiguriert, zumindest die gemessenen Blutzuckerkonzentration als Input zu verwenden, um das aktuelle oder künftige Vorliegen eines Hungergefühlt und/oder den Zeitprunkt des Auftretens des Hungergefühls vorherzusagen. Der vorherzusagende physiologische Zustand ist also ein Zustand, in dem das Kind Hunger hat. Erfindungsgemäß wird ein Hungergefühl vorhergesagt wenn der aktuelle oder künftige Blutzuckerspiegel unterhalb eines vordefinierten Grenzwertes liegt.

Beispielsweise kann die Auswertungssoftware dazu konfiguriert sein, den aktuellen und/oder künftigen Blutzuckerspiegel des Kindes als Funktion der Fließeigenschaften des Blutes zu berechnen und auf Basis des berechneten Blutzuckerspiegels ein aktuell vorliegendes oder künftiges Hungergefühl vorherzusagen.

Die Fließeigenschaften des Blutes hängt unter anderem vom Blutzuckerspiegel ab. Der Blutzuckerspiegel ist annähernd proportional zur Viskosität des Bluts und invers proportional zur Flussrate. Die Auswertungssoftware kann z.B. ein konvolutionales neurales Netzwerk enthalten, welches den Blutzuckerspiegel vom PPG Signal ableiten kann.

Beispielsweise kann die Ableitung mittels der besagten Netzwerke so erfolgen, wie dies in S. Hossain, B. Debnath, S. Biswas, M. J. Al-Hossain, A. Anika and S. K. Zaman Navid, "Estimation of Blood Glucose from PPG Signal Using Convolutional Neural Network," 2019 IEEE International Conference on Biomedical Engineering, Computer and Information Technology for Health (BECITHCON), 2019, pp. 53-58, doi: 10.1109/BECITHCON48839.2019.9063187 beschrieben ist.

Alternativ dazu kann der Blutzuckerwert aus dem PPG Sensorsignal auch gemäß einer Methode abgeleitet werden, wie sie in Delbeck S, et al.: "Non-invasive monitoring of blood glucose using optical methods for skin spectroscopy-opportunities and recent advances", Anal Bioanal Chem. 2019 Jan;411(1):63-77. doi: 10.1007/s00216-018-1395-x. Epub 2018 Oct 3. PMID: 30283998 beschrieben ist: der PPG Sensor führt pulsierende Messungen im kurzwelligen Nahinfrarot-Spektralbereich mit LEDs bei 935, 950 und 1070 nm durch. Die Vorhersage der Glukosekonzentration erfolgte über ein künstliches neuronales Netzwerk (ANN) nach Vorverarbeitung der zeitabhängigen Signale durch einen auf dem neuronalen Netzwerk basierenden adaptiven Rauschunterdrückungsfilter (Adaline). Nach dem Training des neuronalen Netzes wird das Netz zur Vorhersage des Blutzuckerspiegels auf Basis der vom PPG Sensor erfassten spektralen Daten verwendet.

Die Auswertungssoftware kann gemäß Ausführungsformen ein weiteres neuronales Netz oder einen anderen Vorhersagealgorithmus beinhalten oder operativ an diesen gekoppelt sein, wobei das weitere neuronale Netz bzw. der andere Vorhersagealgorithmus dazu ausgebildet ist, das aktuelle oder künftige Hungergefühl als Funktion der berechneten Glukosekonzentration im Blut des Kindes vorherzusagen. Beispielsweise kann es sich bei dem weiteren neuronalen Netz ebenfalls um ein konvolutionales neuronales Netz handeln.

Die korrekte Erkennung bzw. frühzeitige Vorhersage eines Hungergefühls des Kindes kann für die Eltern aus vielen Gründen vorteilhaft und wichtig sein: Kinder können sich noch nicht sprachlich ausdrücken, sodass es für die Eltern oftmals nicht möglich ist, zu erkennen, ob das Weinen eines Kindes durch ein Hungergefühl, eine Verletzung, eine Krankheit oder sonstige Ursache hervorgerufen wird. Durch die Verwendung des tragbaren Geräts, welches das Hungergefühl des Kindes auf Basis eines gemessenen Blutglukosespiegel erkennt bzw. vorhersagt, wird es den Eltern ermöglicht, die Bedürfnisse Ihres Kindes besser zu erkennen.

Ein weiterer Vorteil kann sich daraus ergeben, dass ein Hungergefühl frühzeitig erkannt wird, also bereits zu einem Zeitpunkt, in welchem das Gefühl noch nicht so stark geworden ist, dass das Kind zu weinen beginnt. Dies kann es den Eltern ermöglichen, frühzeitig die Nahrung zuzubereiten bzw., falls die Eltern mit dem Kind auf Reisen sind, frühzeitig eine Lokalität aufzusuchen, in welcher das Kind gefüttert werden kann.

Nach Ausführungsformen umfassen die Sensoren einen photoplethysmograpischen Sensor, hier als PPG Sensor bezeichnet. Die Auswertungssoftware ist dazu ausgebildet, aus den vom PPG Sensor erfassten Signalen neben der Herzfrequenz, der Sauerstoffsättigung, und der Atemfrequenz des Kindes auch die gemessene Blutzuckerkonzentration des Kindes abzuleiten und zumindest die Blutzuckerkonzentration als Input der Auswertungssoftware zur Verfügung zu stellen.

Dies hat den Vorteil, dass die Messung der Blutzuckerkonzentration nicht-invasiv und sehr häufig, z.B. regelmäßig, vorgenommen werden kann, sodass bei einem Absinken des Blutzuckerwertes der Zeitpunkt, ab welchem ein Hungergefühl eintritt bzw. so stark wird, dass das Kind dies durch Weinen anzeigt, vorhergesagt werden kann.

Gemäß mancher Ausführungsformen werden die Blutparameter, die als Korrekturparameter bei der Vorhersage des erhöhten SIDS-Risikos verwendet werden, auch verwendet, um fehlerhafte Blutzuckermessungen zu erkennen.

Erfindungsgemäß ist die Auswertungssoftware direkt oder mittels einer Softwareapplikation des Telekommunikationsgeräts oder über das Server-Computersystem an ein elektronisches Gerät zur Nahrungsvorbereitung kommunikativ gekoppelt. Die Auswertungssoftware und optional auch die Softwareapplikation des Telekommunikationsgeräts ist dazu konfiguriert, in Antwort auf die Vorhersage, dass das Hungergefühl jetzt oder in der Zukunft eintreten wird, das elektronische Gerät zu aktivieren und dadurch das elektronische Gerät zur Vorbereitung der Nahrung für das Kind zu veranlassen.

Beispielsweise kann es sich bei dem elektronischen Gerät oder um einen Milchflaschenwärmer, einen Wasserkocher oder eine Mikrowelle oder dergleichen handeln.

### Weitere Ausführungsformen

Gemäß Ausführungsformen der Erfindung kann die Auswertungssoftware für die Erkennung und/oder Vorhersage einer Vielzahl von physiologischen Zuständen verwendet werden. Neben der Vorhersage eines erhöhten Risikos für den plötzlichen Kindstod und der Vorhersage eines Hungergefühls bzw. der Vorhersage der Zeit des Auftretens des Hungergefühls kann das Gerät zum Beispiel zur Erkennung von Fieber sowie verschiedener akuter oder chronischer Erkrankungen verwendet werden.

Somit können es Ausführungsformen der Erfindung erlauben, Auffälligkeiten zu erkennen, die zum Beispiel ein Hinweis auf angeborene Erkrankungen, akute oder chronische Erkrankungen sein können.

Dank der Vielzahl an Parametern, die von dem tragbaren Gerät erfasst werden können, wird eine sehr breite Datenbasis geschaffen, die eine qualitativ hochwertige Vorhersage bezüglich der aktuellen und künftigen physiologischen Zustände des Kindes erlaubt.

Gemäß manchen Ausführungsformen ist die Auswertungssoftware dazu ausgebildet, selektiv das aktuelle oder künftige Vorliegen eines physiologisch problematischen Zustands des Kindes zu erkennen, der sofortige Intervention erfordert. Ein solcher physiologischer Zustand kann z.B. das erhöhte Risiko für den plötzlichen Kindstod sein. Die Auswertungssoftware leitet zumindest einige der vom tragbaren Gerät gemessenen oder abgeleiteten Vitalparameter oder Vorhersagezwischenergebnisse über ein Netzwerk an das Server-Computersystem weiter, ohne lokal ein Vorhersageendergebnis zu berechnen, um dem Server-Computersystem die Vorhersage physiologischer Zustände zu ermöglichen, die keine sofortige Intervention erfordern.

Dies kann vorteilhaft sein, da die Rechenkapazitäten des tragbaren Geräts aufgrund seiner geringen Größe begrenzt sind.

Dadurch, dass kritische physiologische Zustände durch das Gerät selbst vorhergesagt und nicht-kritische Zustände und/oder die erfassten Rohdaten regelmäßig oder gesammelt ("bulk upload") z.B. im Zuge eines Ladevorgangs an den Server zur serverseitigen Analyse gesendet werden, kann sichergestellt werden, dass unabhängig von der Frage, ob eine Netzwerkverbindung zu dem Server-Computersystem besteht oder nicht immer vom tragbaren Gerät selbst ein Alarmsignal an das Telekommunikationsgerät gesendet werden kann. Auch wird so gewährleistet, dass das Kind in jeder Situation - sei es beim Schlafen auf dem Wohnzimmersofa oder auf Reisen - bezüglich der wirklich kritischen physiologischen Zustände und Parameter (SIDS Risiko, Sauerstoffkonzentration im Blut, etc.) überwacht ist, da die Kernfunktionalität des tragbaren Bändchens unabhängig vom Server-Computersystem und unabhängig von der Verfügbarkeit externer Sensoren immer verfügbar ist. Die Anbindung an das Server-Computersystem und/oder das Heranziehen weiterer Parameter, die von externen Sensoren geliefert werden, können das Vorhersageergebnis noch weiter verfeinern. Die Vorhersagequalität bezüglich kritischer Systemzustände wird daher im Kontext z.B. des üblichen Schlafplatzes des Kindes, wo sich ggf. auch Kamera und Mikrophon und Basisstation befinden, noch etwas exakter sein. Dennoch kann das Kind jederzeit spontan auch an einen anderen Ort gebracht werden, wo weder Netzanschluss noch Kameraüberwachung möglich ist, der Basisschutz bleibt bestehen, solange das Kind das tragbare Gerät am Körper befestigt und die Betreuungspersonen die zugehörige Software auf dem Telekommunikationsgerät instanziiert haben.

Die Batterie des Gerätes wird geschont, sodass das Gerät eine längere Zeit betrieben werden kann, ohne die Batterien zu wechseln oder neu zu laden. Die Verarbeitung der Vorhersageergebnisses zum Versand kostet Rechenleistung, zum Beispiel für die Konvertierung der Daten in das richtige Format für den Versand oder für den Aufbau eines Kommunikationskanals. Dadurch, dass nicht bei jeder Messung und jeder im Anschluss daran vorgenommenen Vorhersage die Messergebnisse bzw. Vorhersageergebnisse versendet werden, sondern die Vorhersageergebnisse nur dann versendet werden, wenn ein kritischer oder problematischer physiologischer Zustand oder Parameterwert erkannt oder vorhergesagt wird, wird also Rechenleistung gespart.

Außerdem wird gemäß manchen Ausführungsformen der Datenverkehr reduziert, denn die Verarbeitung der für die kritischen Zustände relevanten Rohdaten findet bereits auf dem tragbaren Gerät statt, sodass anstatt der Rohdaten nur noch die Vorhersageergebnisse und optional noch einige wenige für das Vorhersageergebnis relevanten Rohdaten übermittelt werden müssen wohingegen die Gesamtheit an Sensordaten oder zumindest die Sensordaten, die für die Vorhersage eines anderen physiologischen Zustand dienen, vorzugsweise gesammelt zu einem späteren Zeitpunkt übermittelt werden.

Beispielsweise kann die Datenübertragung an das Telekommunikationsgerät über ein Funksignal, insbesondere über ein Funksignal gemäß dem Bluetooth Protokoll, erfolgen. Es ist aber auch möglich, dass die Datenübertragung über WLAN erfolgt. Gemäß mancher Ausführungsformen werden zumindest einige Daten vom tragbaren Gerät zunächst an das Server-Computersystem übertragen, z.B. direkt über eine WLAN-Verbindung ins Internet, oder indirekt per z.B. Funk oder WLAN an eine Basisstation und von dieser an das Server-Computersystem.

Nach Ausführungsformen der Erfindung ist die Auswertungssoftware dazu ausgebildet, ein aktuelles oder künftiges Vorliegen eines physiologisch problematischen Zustands des Kindes zu erkennen,
- wenn ein Wert zumindest eines Vitalparameters oder Umgebungsparameters außerhalb eines vorab festgelegten Normbereichs liegt; und
- wenn ein Muster der Werte mehrerer Vitalparameter erkannt wird, welches einen aktuellen oder künftigen problematischen physiologischen Zustand des Kindes anzeigt, wobei das Muster auch erkannt werden kann, wenn alle Vitalparameter und/oder Umgebungsparameter einzeln jeweils innerhalb ihres Normbereichs liegen.

Die Auswertungssoftware ist dazu ausgebildet, in Reaktion auf das Erkennen eines aktuell oder künftig vorliegenden physiologisch problematischen Zustands eine Nachricht bezüglich des vorhergesagten problematischen physiologischen Zustands an das mobile Telekommunikationsgerät zu senden.

Somit sind die Eltern nicht nur gewarnt, wenn z.B. ein erhöhtes SIDS-Risiko festgestellt wird, sondern auch, wenn z.B. der Blutsauerstoffwert unter einen Mindestwert gefallen ist oder wenn die Umgebungstemperatur einen vordefinierten Maximalwert übersteigt.

Nach Ausführungsformen der Erfindung handelt es sich bei dem tragbaren Gerät um ein Armband oder um ein Band am Fußgelenk oder Bein des Kindes. Dies kann vorteilhaft sein, da das Kind in seiner Bewegungsfreiheit nicht eingeschränkt wird, das Gerät an diesen Gliedmaßen sicher befestigt werden kann und vor allem weil es möglich ist, z.B. durch Verwenden eines elastischen Materials in den Bändern oder durch das Einstellen eines Verschlusses einen Anpressdruck einzustellen, sodass die Sensoren mit einem gewissen Mindestdruck am Körper des Kindes anliegen, was die Qualität der Messungen erhöht.

Beispielsweise kann das Band eine Länge haben, die dazu ausgebildet ist, an einem Arm, einem Fußgelenk oder einem Bein mit dem Umfang von ca. 7-15 cm (entsprechend dem Umfang der entsprechenden Gliedmaßen bei Babys und Kleinkindern) getragen zu werden. Beispielsweise kann das Band einschließlich des Verschlussmechanismus 7,5 bis 20 cm lang sein.

Nach Ausführungsformen umfassen die Sensoren einen oder mehrere Drucksensoren, die dazu ausgebildet sind, den Anpressdruck des Gerätes am Körper des Kindes zu erfassen. Die Auswertungssoftware ist dazu ausgebildet, auf Basis des gemessenen Anpressdrucks zu erkennen, ob der Anpressdruck innerhalb eines vordefinierten zulässigen Anpressruckbereichs ist, innerhalb welchem die ein oder mehreren Sensoren zur Erfassung der Vitalwerte korrekt arbeiten können und innerhalb welchem das Band beim Kind keine Druckschmerzen erzeugen wird. Die Auswertungssoftware ist dazu ausgebildet, eine Warnung über ein Signalelement des Geräts an den Nutzer und/oder über die Schnittstelle an das Telekommunikationsgerät auszugeben, falls der gemessene Anpressruck außerhalb des zulässigen Anpressdruckbereichs ist. Zusätzlich oder alternativ dazu ist die Auswertungssoftware dazu ausgebildet, eine Messung von Vitalparametern durch die ein oder mehreren Sensoren so lange zu unterbinden, bis der Anpressdruck wieder innerhalb des zulässigen Anpressruckbereichs ist.

Dies kann vorteilhaft sein, weil hierdurch sichergestellt wird, dass die Sensoren immer in ausreichendem Kontakt zum Körper sind, um aussagekräftige Messungen vornehmen zu können. Dies reduziert die Anzahl falscher Vorhersagen und verhindert, dass Messdaten aufgezeichnet werden, die wegen fehlendem Kontakt zum Körper keinerlei Aussagekraft haben.

Das tragbare Gerät kann z.B. eine Warnung bezüglich des fehlenden Kontakts aussenden. Beispielsweise kann die Warnung direkt ausgegeben werden über einen Lautsprecher, der in das tragbare Gerät integriert ist, oder über eine Lichtquelle, zum Beispiel eine LED Lampe, die aufleuchtet oder blinkt. Zusätzlich oder alternativ dazu kann das tragbare Gerät die Warnung aber auch an eine Software auf dem tragbaren Telekommunikationsgerät senden oder an eine Basisstation, sodass die Warnung bezüglich des fehlenden Kontakts von dem Telekommunikationsgerät und/oder der Basisstation ausgegeben wird.

Dadurch, dass das tragbare Gerät eine Warnung an den Nutzer ausgibt, kann dieser das Gerät neu an geeigneter Position am Körper des Kindes anbringen.

Nach Ausführungsformen der Erfindung ist das tragbare Gerät dazu konfiguriert, die gemessenen Vitalparameter und optional auch den zumindest einen Umgebungsparameter automatisch und regelmäßig zu erfassen und mit der Auswertungssoftware auszuwerten. Die Daten können dann gesammelt und z.B. per Push- oder Pull-Funktionalität an den Server-Computer hochgeladen und/oder an das Telekommunikationsgerät übermittelt werden. Beispielsweise kann die Übermittlung erfolgen, wenn das tragbare Gerät geladen wird und/oder wenn die Betreuungspersonen eine Anfrage bezüglich der aktuellen Daten über das Telekommunikationsgerät an das tragbare Gerät senden.

Gemäß Ausführungsformen ist die Schnittstelle zur Übertragung von Daten an das Telekommunikationsgerät eine Schnittstelle zur Datenübertragung über ein Nahfeldsignal, insbesondere über ein Funksignal, insbesondere eine Bluetooth-Schnittstelle.

Das tragbare Gerät ist dazu konfiguriert, in einem strahlungsarmen und in einem strahlungsnormalen Betriebszustand betreibbar zu sein.

Das tragbare Gerät dazu konfiguriert ist, im normalen Betriebsmodus, wenn kein physiologischer Zustand vorhergesagt und wenn kein Vital- oder Umgebungsparameter gemessen wird, der eine sofortige Intervention erfordert, im strahlungsarmen Betriebszustand zu arbeiten. Falls die Auswertungssoftware das aktuelle oder künftige Vorliegen eines physiologisch problematischen Zustands, insbesondere ein erhöhtes Risiko für einen plötzlichen Kindstod und/oder ein Hungergefühl, oder das Vorliegen eines Vital- oder Umweltparameters in einem gesundheitskritischen Wertebereich feststellt, wechselt das tragbare Gerät automatisch in den strahlungsnormalen Betriebsmodus. Vorzugsweise kehrt das Gerät nach Übermittlung der relevanten Daten bezüglich des kritischen physiologischen Zustands und/oder Parameters automatisch in den strahlungsarmen Zustand zurück.

Dies schont nicht nur die Batterie des tragbaren Geräts, sondern minimiert auch die Funkstrahlung, die von manchen Eltern als problematisch angesehen wird.

Je nach verwendeter Technologie kann der strahlungsarme Betriebsmodus leicht unterschiedlich implementiert sein.

Bei der Verwendung von Bluetooth als Nahfeld-Kommunikationstechnologie kann der Wechsel des tragbaren Geräts in den strahlungsarmen Betriebsmodus z.B. wie folgt implementiert sein:

### Option 1: Wechsel der "Advertising-Rate" innerhalb des Advertising Betriebsmodus

In dieser Variante arbeitet das tragbare Gerät sowohl im Normalbetriebsmodus als auch im strahlungsarme Betriebsmodus im sog. "Advertising" Modus. In diesem Arbeitsmodus von Bluetooth-Geräten ist das betreffende Gerät nicht mit weiteren Geräten dauerhaft verbunden (aneinander gekoppelt). Das tragbare Gerät und das Telekommunikationsgerät sind in diesem Betriebsmodus also nicht gekoppelt und es findet keine Übertragung der von den Sensoren erfassten Daten vom tragbaren Gerät an das Telekommunikationsgerät statt. Im "Advertising" Zustand sendet das tragbare Gerät in regelmäßigen Abständen z.B. 10 Sekunden oder 1 Minute, ein sog. "Advertising" Datenpaket per Funk aus mit der Information, dass das tragbare Gerät existiert, dass es jedoch keine Verbindung mit dem Telekommunikationsgerät aufbauen möchte. Die Rate des Versands dieser "Advertising"-Datenpakete wird als "Advertising-Rate" bezeichnet. Ein "strahlungsarmer Betriebszustand" ist hier ein "Advertising"-Zustand eines Bluetooth fähigen Gerätes, in welchem die Advertising-Rate unterhalb eines vordefinierten Maximalwerts liegt, z.B. maximal ein Advertising-Datenpaket pro Minute oder maximal ein Advertising-Datenpaket pro 10 Sekunden. Ein "strahlungsnormaler Betriebszustand" ist hier ein "Advertising"-Zustand eines Bluetooth fähigen Gerätes, in welchem die Advertising-Rate oberhalb des vordefinierten Maximalwerts liegt, z.B. mehr als ein Advertising-Datenpaket pro Minute oder mehr als ein Advertising-Datenpaket pro 10 Sekunden.

Innerhalb dieses "Advertising"-Betriebsmodus befindet sich das tragbare Gerät im Normalfall, wenn kein kritischer physiologischer Zustand vorhergesagt wird, in dem strahlungsarmen Betriebsmodus. In diesem werden die Vitalparameter lokal im tragbaren Gerät gespeichert und analysiert und mit geringer Frequenz ein Advertising-Datenpaket versendet, der im Wesentlichen nur beinhaltet, dass das tragbare Gerät existiert aber keine Kopplung an andere Geräte wünscht.

Sobald die Auswertungssoftware des tragbaren Geräts einen kritischen physiologischen Zustand vorhersagt, der eine sofortige Intervention erfordert, erhöht die Auswertungssoftware die Frequenz des Aussendens der "Advertising"-Datenpakete, damit das Datenpaket möglichst schnell übermittelt werden kann. Vorzugsweise wechselt das tragbare Gerät nach Übermittlung eines oder mehrerer Datenpakete, die einen Alarm und/oder Messwerte bezüglich des vorhergesagten kritischen physiologischen Zustands und/oder der kritischen Vital- oder Umgebungsparameter enthalten, von dem "strahlungsarmen" in den "strahlungsnormalen" Betriebsmodus. Nach Versand des Alarm-Datenpakets bzw. der Alarm-Datenpakete kehrt das tragbare Gerät und dessen Funkmodul wieder in den strahlungsarmen Betriebsmodus zurück.

Alle Geräte in Reichweite dieses Advertising-Datenpakets, die bereits einmal mit dem tragbaren Gerät verbunden waren (die mit diesem gekoppelt waren), insbesondere alle Telekommunikationsgeräte für die dies zutrifft, können dieses Datenpaket empfangen und weiterverarbeiten und ggf. dem Nutzer auf dem Display des Telekommunikationsgeräts anzeigen. Für eine höhere Gewissheit, dass die Alarmmeldung beim Telekommunikationsgerät ankommt, kann die Advertising Anfrage zusätzlich die Information enthalten, dass das tragbare Gerät sich nun mit dem Telekommunikationsgerät verbinden (koppeln) möchte. Sobald diese Verbindung hergestellt ist, ist für das tragbare Gerät auch erkennbar, dass das Datenpaket mit dem Alarm beim Empfänger angekommen ist.

### Option 2: Wechsel der "Rückmelde-Rate" innerhalb eines gekoppelten Betriebsmodus

Gemäß dieser Implementierungsvariante ist das tragbare Gerät im normalen Betriebsmodus an das Telekommunikationsgerät einer Betreuungsperson gekoppelt, es besteht also eine aktive Verbindung zwischen dem tragbaren Gerät und dem Telekommunikationsgerät. Normalerweise senden Bluetooth-Geräte im gekoppelten Betriebszustand sehr häufig Anfragen (z.B. ca. 100 mal pro sec), ob das verbundene Gerät noch da ist und erwartet vom verbundenen Gerät die Rückmeldung, dass dies der Fall ist.

Im strahlungsarmen Betriebsmodus, wenn das tragbare Gerät also keinen interventionsbedürftigen physiologischen Zustand vorhersagt bzw. interventionsbedürftigen Umgebungs- oder Vitalparameter misst, arbeitet das tragbare Gerät in einem strahlungsarmen Betriebsmodus, in welchem das tragbare Gerät zwar an das Telekommunikationsgerät gekoppelt ist, aber an dieses zurückmeldet, dass es sich für eine vordefinierte Anzahl weiterer Rückmeldungen (z.B. die nächsten 100 Rückmeldungen) nicht melden wird. Dadurch werden weniger Rückmeldungs-Datenpakete vom tragbaren Gerät an das Telekommunikationsgerät gesendet. Wird dagegen ein kritischer physiologischer Zustand oder Vitalparameter oder Umgebungsparameter vorhergesagt bzw. gemessen, sendet das tragbare Gerät sofort und ohne auf den Ablauf der "abgesagten" Rückmeldungszyklen zu warten eine Rückmeldung mit Informationen bezüglich des interventionsbedürftigen Zustands an das gekoppelte Telekommunikationsgerät. Dabei geht das tragbare Kommunikationsgerät zunächst in den strahlungsnormalen Betriebsmodus über, da mit der für den gekoppelten Bluetooth-Zustand üblichen Frequenz Rückmeldungen gesendet werden. Sobald aber die Warnung mit Informationen bezüglich des interventionsbedürftigen Zustands an das gekoppelte Telekommunikationsgerät übertragen wurde, kehrt das tragbare Gerät automatisch wieder in den strahlungsarmen Betriebsmodus zurück, indem es zurückmeldet, dass es sich für eine vordefinierte Anzahl weiterer Rückmeldungen nicht melden wird. Bei der vordefinierten Anzahl weiterer Rückmeldungen handelt es sich vorzugsweise um mehr als 50, weiterhin vorzugsweise um mehr als 100 Rückmeldungen.

### Option 3: Reduktion der Sendeleistung bei guter Verbindung

Gemäß einer dritten Implementierungsvariante ist das tragbare Gerät im normalen Betriebsmodus an das Telekommunikationsgerät gekoppelt und ermittelt kontinuierlich die Qualität der Verbindung. Beispielsweise wird ermittelt, wie häufig eine erwartete Rückkopplungs-Nachricht ausbleibt oder wie hoch die Signalstärke des Bluetooth-Signals des Telekommunikationsgeräts ist.

Wenn aktuell keine kritischen Systemzustände vorhergesagt wurden und keine kritischen Umgebungs- oder Vitalparameter erfasst oder berechnet wurden, und wenn außerdem die Verbindungsqualität zum Telekommunikationsgerät über einem vordefinierten Mindestqualitätsmaß liegt, reduziert das tragbare Gerät die Sendeleistung des Bluetooth-Funkmoduls und geht dadurch in den strahlungsarmen Betriebsmodus über. Wenn die Verbindungsqualität schlecht ist, also unterhalb des vordefinierten Mindestqualitätsmaßes liegt, oder wenn ein physiologischer Zustand vorhergesagt oder ein Umgebungs- oder Vitalparameter erfasst wurde, der eine sofortige Intervention erfordert, behält das Funkmodul des tragbaren Geräts die Sendeleistung des Funkmoduls bei oder erhöht diese.

Hierdurch kann Energie gespart und die Lebensdauer der Batterie erhöht werden.

Neben Bluetooth können auch andere Standards und/oder Protokolle für den nahfeldbasierten Datenaustausch verwendet werden, z.B. ZigBee.

Bei der Verwendung von z.B. Bluetooth oder ZigBee besitzt das tragbare Gerät ein FunkModul ("Übertragungsmodul"). Liegen keine Auffälligkeiten vor, die die unmittelbare Aufmerksamkeit der Betreuungsperson erfordern, werden die Daten lokal gespeichert und das Übertragungsmodul wird in einem strahlungsarmen Betriebsmodus betrieben. In diesem Zustand werden das tragbare Gerät und das Empfängergerät (also das portable Telekommunikationsgerät sowie optional auch die Basisstation), gemäß Ausführungsformen nicht kontinuierlich synchronisiert. Wird mittels der Auswertungssoftware im tragbaren Gerät festgestellt, dass eine Auffälligkeit und/oder kritischer physiologischer Zustand vorliegt, wird das Funkmodul in den strahlungsnormalen Betriebsmodus versetzt und es werden Daten (Messwerte und/oder Vorhersageergebnisse) an das Empfängergerät gesendet.

Da die Reichweite eines Bluetooth-Signals oder ZigBee-Signals in vielen Haushalten oft geringer ist als z.B. das WLAN-Signal, können alternativ zu Bluetooth bzw. ZigBee auch das WLAN bzw. eine sonstige geeignete internetbasierte Datenkommunikation zwischen Auswertungssoftware und Telekommunikationsgerät implementiert werden.

Nach Ausführungsformen umfasst das tragbare Gerät ein oder mehrere Umgebungsparameter-Sensoren ausgewählt aus einer Gruppe beinhaltend:
- ein Thermometer zum Messen der Umgebungstemperatur;
- ein Messgerät zum Messen der Umgebungsluftfeuchte;
- Gase, insbesondere CO₂;
- ein Mikrophon zum Erfassen von Umgebungsgeräuschen und/oder von Geräuschen des Kindes;
- UV-Sensor zur Erfassung einer kumulativen UV-Strahlendosis, insbesondere einer täglichen kumulativen UV-Strahlendosis.

Nach Ausführungsformen umfassen die Sensoren zur Erfassung der Vitalparameter weitere Sensoren ausgewählt aus einer Gruppe beinhaltend:
- Beschleunigungssensor, z.B. zur Erkennung der Lage (insb. Bauchlage oder Rückenlage) des Kindes;
- Thermometer zur Messung der Hauttemperatur des Kindes;
- Videokamera (insb. Wärmebildkamera zur Erkennung einer Bauchlage oder Rückenlage);

In einem weiteren Aspekt betrifft die Erfindung ein System beinhaltend das Gerät und ein oder mehrere der folgenden weiteren Komponenten:
- das portable Telekommunikationsgerät, wobei auf dem portablen Telekommunikationsgerät eine Nutzersoftware instanziiert ist, wobei die Nutzersoftware mit der Auswertungssoftware interoperabel ist und dazu ausgebildet ist, die von dem tragbaren Gerät über die Schnittstelle empfangenen Vorhersageergebnisse dem Nutzer anzuzeigen und/oder dem Nutzer die Konfiguration der Auswertungssoftware zu ermöglichen; und/oder
- das Server-Computersystem; und/oder
- eine Basisstation, an welche ein oder mehrere externe Sensoren zur Messung von Vitalparametern des Kindes oder von Umgebungsparametern der Umgebung des Kindes gekoppelt sind; die Basisstation ist dazu ausgebildet, die von den externen Sensoren gemessenen Parameterwerte an das Serversystem in ursprünglicher oder verarbeiteter Form weiterzuleiten. Beispielsweise können die an die Basisstation kommunikativ gekoppelten oder koppelbaren externen Sensoren eine Videokamera, insbesondere eine Wärmebildkamera, ein Mikrophon, einen Umgebungstemperatursensor, einen Sensor für die Luftfeuchtigkeit und/oder einen Sensor zur Erfassung der CO₂ Konzentration der Umgebungsluft umfassen. Gemäß manchen Ausführungsformen enthält die Basisstation ein Modul zum Laden des tragbaren Geräts, z.B. über ein Induktionsfeld. Gemäß manchen Ausführungsformen enthält die Basisstation eine Netzwerkschnittstelle, insbesondere eine WIFI Schnittstelle, zur kommunikativen Kopplung der Basisstation und/oder der an diese kommunikativ gekoppelten externen Sensoren an das Server-Computersystem. Die Basisstation enthält auch eine Software, die mit der Auswertungssoftware auf dem tragbaren Gerät wie auch mit der Server-Applikation interoperabel ist; und/oder
- ein oder mehrere der externen Sensoren, insbesondere eine Videokamera, insbesondere eine Wärmebildvideokamera.

Nach Ausführungsformen der Erfindung beinhaltet die Vorhersagesoftware zumindest ein prädiktives Modell zur Vorhersage des zumindest einen physiologischen Zustands. Das zumindest eine prädiktive Modell ist ein durch ein maschinelles Lernverfahren auf Basis eines Trainingsdatensatzes erzeugtes Modell. Insbesondere kann es sich bei dem prädiktiven Modell um ein neuronales Netz handeln. Neuronale Netze haben sich als besonders geeignet erwiesen, die Zusammenhänge verschiedener Vitalparameter und/oder Umgebungsparameter sowie verschiedener physiologischer Zustände zu erfassen und vorherzusagen.

Auf maschinellem Lernen basierende prädiktive Modelle erlauben es, komplexe Abhängigkeiten der Parameter untereinander wie auch mit dem vorherzusagenden physiologischen Zustand zu erkennen und bei der Vorhersage zu berücksichtigen. Gerade im Bereich der Physiologie wirken Vitalparameter und Umgebungsparameter oft auf komplexe und nicht-lineare Weise zusammen, verstärken sich oder schwächen einander ab. Verfahren des maschinellen Lernens, z.B. neuronale Netze, sind in der Lage, diese komplexen Parameterabhängigkeiten zu erfassen und so bei der Vorhersage zu nutzen, dass ggf. nicht nur aktuelle physiologische Zustände, sondern auch in der Zukunft voraussichtlich eintretende Zustände (und ggf. auch der Zeitpunkt des Eintretens) vorhergesagt werden kann.

In einem weiteren Aspekt betrifft die Erfindung ein Verfahren zur Bereitstellung eines tragbaren Geräts zur Überwachung des physiologischen Zustands eines Kindes nach Anspruch 16. Das Verfahren umfasst ein Bereitstellen eines Trainingsdatensatzes. Der Trainingsdatensatz beinhaltet mehrere Datensätze. In jedem Datensatz ist zumindest ein physiologischer Zustand des Kindes spezifiziert, der verknüpft gespeichert ist mit Vitalparametern des Kindes (insbesondere Herzfrequenz, Sauerstoffsättigung und Atemfrequenz, ggf. auch Hauttemperatur oder abgeleitete Körperkerntemperatur, Bewegungsmuster, Video- oder Audiodaten etc.). Optional kann der Datensatz auch ein oder mehrere Umgebungsparameter enthalten, z.B. Umgebungstemperatur, Luftfeuchtigkeit, CO₂ Konzentration der Umgebungsluft etc. Vorzugsweise enthält der Datensatz für jeden der Vitalparameter und/oder Umgebungsparameter eine Vielzahl von Datenwerten, die jeweils verknüpft gespeichert sind mit einem Zeitstempel, wobei auch der physiologische Zustand verknüpft mit einen Zeitstempel gespeichert ist. Dies erlaubt es, nicht nur Korrelationen zwischen mehreren Parametern und physiologischen Zuständen zu erkennen, sondern auch deren zeitliche Abhängigkeiten.

Das Verfahren umfasst ferner ein Durchführen eines maschinellen Lernverfahrens auf den Trainingsdaten zur Erzeugung des zumindest einen prädiktiven Modells. Das zumindest eine prädiktive Modell ist dazu ausgebildet, den aktuellen oder künftigen physiologischen Zustands des Kindes auf Basis zumindest der Herzfrequenz, der Sauerstoffsättigung, und der Atemfrequenz sowie optional weiteren Vitalparametern und/oder Umgebungsparametern vorherzusagen. Gemäß mancher Ausführungsformen lernt das prädiktive Modell auch zeitliche Abhängigkeiten, sodass es in der Lage ist auch den Zeitpunkt des Eintretens des physiologischen Zustands bei einer gegebenen Menge an Parameterwerten vorherzusagen.

Das Verfahren umfasst ferner eine Installation einer Auswertungssoftware, die das zumindest eine prädiktive Modell beinhaltet, auf dem tragbaren Gerät. Das Gerät ist zum Tragen am Körper eines Kindes ausgebildet, wobei das Gerät so dimensioniert und geformt ist, dass es von einem Baby oder Kleinkind getragen werden kann.

Das Gerät umfasst einen oder mehrere Sensoren zur Erfassung von mehreren Vitalparametern des Kindes. Die Vitalparameter umfassen zumindest die Herzfrequenz, die Sauerstoffsättigung und die Atemfrequenz. Die Auswertungssoftware ist dazu ausgebildet, das zumindest eine prädiktive Modell zur Vorhersage des physiologischen Zustands auf Basis der von den Sensoren erfassten Messwerten zu verwenden.

Das tragbare Gerät umfasst ferner eine Schnittstelle zur Übertragung eines Vorhersageergebnisses bezüglich des physiologischen Zustands an ein mobiles Telekommunikationsgerät eines Nutzers (einer Betreuungsperson, z.B. ein Elternteil) und/oder an ein Server-Computersystem.

Die Schnittstelle zur Kommunikation mit dem Telekommunikationsgerät ist vorzugsweise eine Schnittstelle zur Nahfeldkommunikation, z.B. per Funk oder WLAN, gemäß mancher Ausführungsformen kann die Schnittstelle aber auch eine Mobilfunkverbindung sein.

Die Schnittstelle zur Kommunikation mit dem Server-Computersystem kann z.B. eine WLAN-Verbindung sein oder eine Mobilfunkverbindung.

Gemäß Ausführungsformen der Erfindung umfasst das zumindest eine Modell ein SIDS-Modell zur Vorhersage eines aktuellen oder künftigen erhöhten Risikos am plötzlichen Kindstod zu sterben. Optional kann das zumindest eine Modell ein oder mehrere weitere prädiktive Modelle enthalten, z.B. ein Hunger-Modell zur Vorhersage ob und/der wann bei dem Kind ein Hungergefühl eintreten wird.

Das SIDS-Modell wird in der Trainingsphase auf Trainingsdaten trainiert, welche zumindest die Sauerstoffkonzentration, die Herzfrequenz und die Atemfrequenz umfassen. Vorzugsweise umfassen die Trainingsdaten neben der Sauerstoffkonzentration noch einen oder mehrere weitere Blutparameter, die als Kontrollparameter dienen, also z.B. CO₂ Konzentration, Methämoglobin und/oder Carboxyhämoglobin. Die Blutparameter der Trainingsdaten werden vorzugsweise unter Realbedingungen erfasst, das heißt, dass auch in den Trainingsdaten Sauerstoffkonzentrationen im Blut enthalten sind, die wegen Messfehlern zu niedrig sind, und die in den Trainingsdaten als fehlerhaft annotiert sind.

Der Trainingsdatensatz für das Training des Hungermodells enthält vorzugweise eine Vielzahl von Datensätzen, die jeweils neben dem Zeitpunkt des Eintretens eines Hungergefühls noch einige weitere mit Zeitstempeln versehene Parameter umfasst, z.B. den Blutzuckerspiegel.

Unter einem "**tragbaren Gerät**" wird hier ein elektronisches Gerät verstanden, welches während der Anwendung am Körper des Benutzers getragen wird. Sie werden auch als "Wearables" bezeichnet. Beispielsweise kann das Gerät mittels bestimmter Befestigungsmittel (z.B. Band, insb. Klettverschluss, Schnallverschluss, Magnetverschluss, etc.) am Körper befestigt oder in die Kleidung integriert sein. Vorzugsweise umfasst das Gerät ein oder mehrere Sensoren und eine Datenverarbeitungseinheit.

Unter einem "**Telekommunikationsgerät**" wird hier jegliches portable und zur Datenübertragung über ein Netzwerk fähige Datenverarbeitungsgerät verstanden, insbesondere ein Mobilfunkgerät, ein Smartphone, eine Smartwatch oder ein Tablet-Computer oder Notebook.

Unter einem "**Kind**" wird hier ein Kleinkind oder Baby verstanden. Unter einem "**Kleinkind**" wird hier ein Kind im zweiten, dritten oder vierten Lebensjahr verstanden, unter einem "Baby" ein Kind im ersten Lebensjahr.

Unter einer "**Batterie**" wird hier eine nicht wiederaufladbare Primärbatterie oder eine wiederaufladbare Sekundärbatterie (landläufig Akkumulator genannt) verstanden.

Unter einem "**prädiktiven Modell**" wird hier eine ausführbare Datei, ein Parametersatz und/oder eine Datenstruktur verstanden, welche es einem Softwareprogramm ermöglicht oder selbst dazu ausgebildet ist, das aktuelle Vorliegen eines bestimmten physischen Zustands einer Entität zu erkennen und/oder das künftige Vorliegen dieses Zustands vorherzusagen. Typischerweise verwendet ein prädiktives Modell historische Daten bezüglich des zu erkennenden bzw. vorherzusagenden Zustands für die Berechnung. Beispielsweise können die historischen Daten als Trainingsdaten verwendet werden, um das in diesen Daten enthaltene Wissen im Zuge eines maschinellen Lernprozesses zu extrahieren und in dem prädiktiven Modell zu speichern. Das Wissen kann insbesondere das Wissen um Korrelationen von Parametern umfassen.

Unter "**Maschinellem Lernen**" wird hier ein Prozess verstanden, durch welchen Wissen über die Zusammenhänge von mehreren Parametern, das in Trainingsdaten enthalten ist, überführt wird in ein sog. "Modell", welches dazu genutzt werden kann, Vorhersagen bezüglich der Eigenschaften von Entitäten und Prozessen automatisch zu berechnen. Das heißt, es werden nicht einfach die Beispiele auswendig gelernt, sondern Muster und Gesetzmäßigkeiten in den Lerndaten erkannt. So kann das System auch unbekannte Daten beurteilen (Lerntransfer). Beispielsweise kann es sich bei dem erzeugten Modell um ein prädiktives Modell in Form eines trainierten künstlichen neuronalen Netzes handeln, oder um andere Datenstrukturen wie z.B. Supportvektormaschinen.

Unter einem **"Vitalparameter"** wird hier ein Datenwert, insbesondere ein numerischer Wert, verstanden, welcher einen Zustand und/oder eine aktuell vorliegende Eigenschaft des Körpers eines Menschen widerspiegelt. Bei einem Vitalparameter kann es sich um einen Datenwert bzw. Rohdatenwert handeln, der als Messwert unmittelbar durch eine Messung gewonnen wird, oder um einen aus gemessenen Rohdaten rechnerisch abgeleiteten Wert.

Unter einem **"Umgebungsparameter"** wird hier wird hier ein Datenwert, insbesondere ein numerischer Wert, verstanden, welcher vollständig oder zumindest größtenteils von Entitäten außerhalb des Körpers eines Menschen abhängen. So ist z.B. die Stärke der UV-Strahlung der Sonne ein Umgebungsparameter, aber auch die Zimmertemperatur, da eine gewisse Erwärmung eines Raumes durch die Körperwärme eines Menschen zwar möglich ist, der Effekt jedoch im Allgemeinen vernachlässigbar ist. Bei einem Umgebungsparameterwert kann es sich um einen Datenwert bzw. Rohdatenwert handeln, der als Messwert unmittelbar durch eine Messung gewonnen wird, oder um einen aus gemessenen Rohdaten rechnerisch abgeleiteten Wert.

Unter einem **"physiologischen Zustand"** wird hier ein biophysikalischer Zustand von bestimmten Lebensvorgängen eines Organismus, z.B. eines Kindes, bezeichnet. Bei dem Zustand kann es sich z.B. um einen gesunden Zustand, einen krankhaften Zustand oder einen risikobehafteten Zustand handeln. Beispielsweise wird ein Zustand, in welchem alle Vitalparameter im Normbereich sind, in der Regel als gesund angesehen, und ein Zustand, bei dem ein oder mehrere wichtige biophysikalische Parameter vom Normbereich abweichen und aktuelle Beschwerden hervorrufen, als krankhafter Zustand bezeichnet. Ein "risikobehafteter" Zustand ist ein solcher, bei welchem aktuell noch keine gesundheitliche Beeinträchtigung der betroffenen Person spürbar ist, bei welchem jedoch das Risiko, dass ein krankhafter Zustand eintreten wird, deutlich erhöht ist.

### Kurze Beschreibung der Zeichnung

Nachfolgend werden Ausführungsformen der Erfindung mit Bezug auf die Zeichnung beschrieben. In der Zeichnung zeigt
- Fig. 1: ein Verfahren zur Bereitstellung eines tragbaren Geräts zur Vorhersage eines physiologischen Zustands eines Kindes;
- Fig. 2: eine Illustration einer als Armband ausgebildeten Variante des Geräts;
- Fig. 3: eine Illustration der Übermittlung eines Alarms per Nahfeldsignal vom Armband an ein Smartphone;
- Fig. 4: ein Blockdiagramm eines Bereichs eines tragbaren Geräts mit mehreren Sensoren;
- Fig. 5: ein Diagramm bezüglich der Ableitung und Verwendung verschiedener Parameter für die Vorhersage eines erhöhten SIDS-Risikos;
- Fig. 6: ein Diagramm bezüglich der Ableitung und Verwendung eines Parameters für die Vorhersage von Hungergefühlen;
- Fig. 7: ein System zur Überwachung der Gesundheit eines Kindes mit mehreren Komponenten.

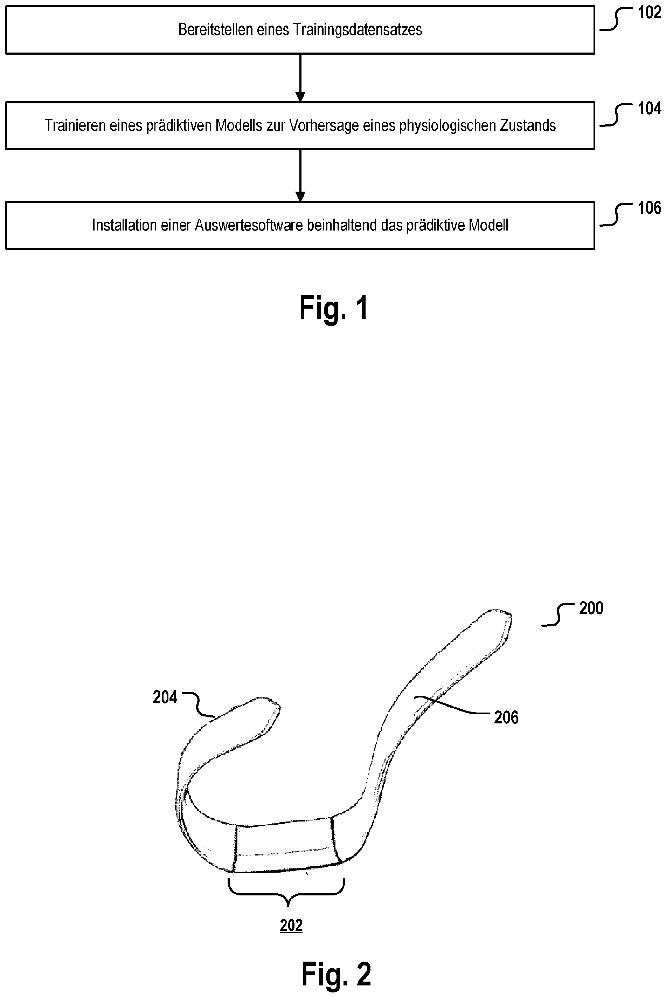

**Figur 1** zeigt Verfahren zur Bereitstellung eines tragbaren Geräts zur Vorhersage eines physiologischen Zustands eines Kindes.

In einem ersten Schritt 102 wird ein Trainingsdatensatz bereitgestellt. Beispielsweise kann der Trainingsdatensatz auf einem Speichermedium bereitgestellt werden oder über ein Netzwerk heruntergeladen werden.

Der Trainingsdatensatz kann z.B. dadurch erzeugt worden sein, dass ein tragbares Gerät mit mehreren Sensoren an mehreren Kleinkindern und Babys angebracht wurde, um mehrere Vitalparameter und/oder Umgebungsparameter über einen längeren Zeitraum hinweg zu erfassen und zu speichern. Außerdem werden die so gewonnenen Daten mit verifizierten physiologischen Zuständen annotiert. Wenn die Zahl der Kinder hinreichend groß ist und der Beobachtungszeitraum hinreichend lang, werden verschiedene, teilweise auch kritische Situationen und dazu korrespondierende physiologische Zustände auftreten. Beispielsweise können Erkältungskrankheiten und damit einhergehend Fieber auftreten. Es kann kurzfristig zu Hungergefühlen kommen, falls es auf einer Reise zu Verzögerungen kommt und die Fütterungszeiten des Kindes nicht eingehalten werden können. Es können auch auffällige Atemmuster (z.B. Apnoen), Hypoxämien und Bradykardien auftreten. Diese und weitere Auffälligkeiten können insbesondere bei Frühgeborenen auftreten und als Trainingsdatensatz erhoben und gespeichert werden. Die Erzeugung des Trainingsdatensatzes kann auch vorsehen, dass neben dem tragbaren Gerät auch externe Sensoren verwendet werden, um zusätzliche Vitalparameter und/oder Umgebungsparameter zu erfassen, um den Trainingsdatensatz zu vergrößern.

Im nächsten Schritt 104 erfolgt das Trainieren eines prädiktiven Modells mithilfe eines maschinellen Lernverfahrens. Es können verschiedene Verfahren wie zum Beispiel neuronale Netzwerke, Supportvektormaschinen und ähnliche Verfahren verwendet werden. Neuronale Netzwerke haben sich jedoch als besonders vorteilhaft in diesem Kontext erwiesen. Die Gesamtheit der Parameter und deren jeweiligen Zeitstempel stellen die Eingangsparameter des zu trainieren den Models dar. Die annotierten physiologischen Zustände des Kindes stellen die Ausgabedaten dar. Im Zuge des Trainings werden verschiedene Parameter des Modells, zum Beispiel Gewichte von Knoten neuronaler Netzwerke, so angepasst, dass die von dem Modell auf Basis eines Satzes von Eingabeparametern vorhergesagte Ausgabe (physiologischer Zustand) möglichst identisch oder ähnlich ist zu den physiologischen Zuständen, die tatsächlich beobachtet und im Trainingsdatensatz annotiert wurden. Dieser Vorgang kann eine Minimierung einer sogenannten "Loss-Funktion" umfassen.

In einem weiteren Schritt 106 kann das trainierte prädiktive Modell in eine Auswertungssoftware integriert und diese auf ein oder mehreren tragbaren Geräten und/oder dem Server-Computersystem installiert werden. Eine mit der Auswertungssoftware interoperable Softwareanwendung kann als sogenannte "App" über den Appstore des jeweiligen Betriebssystemanbieters des Telekommunikationsgeräts zum Download und zur Installation auf dem Telekommunikationsgerät bereitgestellt werden.

**Figur 2** zeigt eine Illustration einer als Armband ausgebildeten Variante des tragbaren Geräts 200. In der hier dargestellten Variante befinden sich alle oder die Mehrzahl der Sensoren innerhalb eines zentralen Sensorblocks 202, an welchem zwei Arme 204, 206 befestigt sind. Größe, Form und Material des Geräts sind dazu ausgebildet, dass es an den Hand- oder Fußgelenken bzw. Armen oder Beinen eines Babys oder Kleinkindes getragen werden kann. Beispielsweise können die Arme aus einem flexiblen Material wie zum Beispiel Silikon oder Stoff gefertigt sein. Sie verfügen über einen Verschlussmechanismus, der es erlaubt, das Gerät 200 sicher am Körper des Kindes zu befestigen. Vorzugsweise ist das Material der Arme 204, 206 elastisch, um einen hinreichenden Anpressdruck zu gewährleisten.

In anderen Ausführungsformen können die Sensoren jedoch auch über eine der beiden Arme oder über beide Arme verteilt sein.

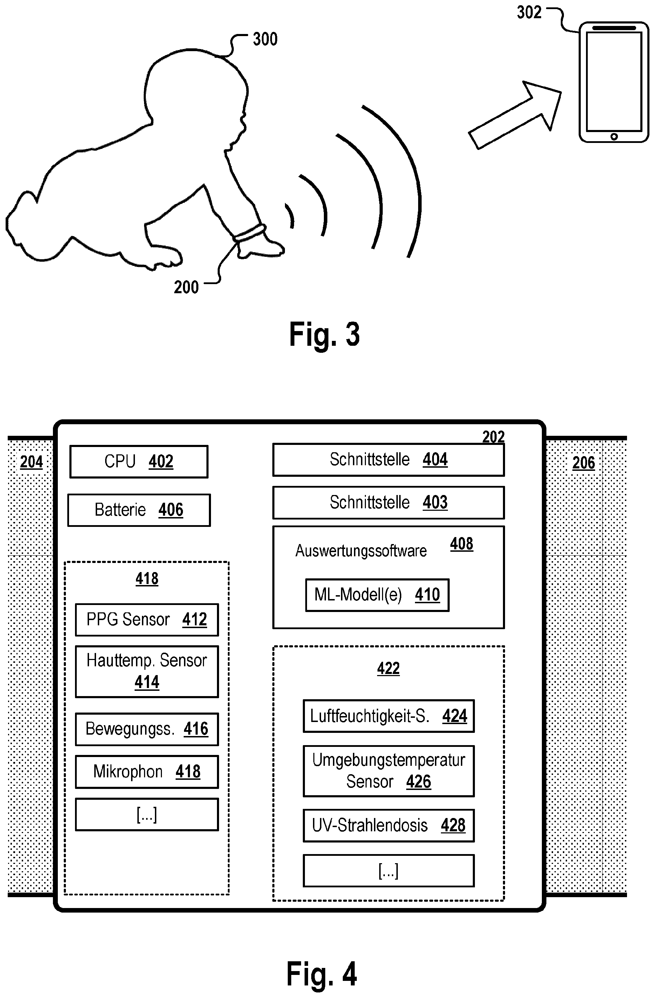

**Figur 3** zeigt eine Illustration der Übermittlung eines Alarms per Nahfeldsignal vom Armband an ein Smartphone. Beispielsweise kann das Armband 200 über ein Modul zur Nahfeld-Kommunikation verfügen, zum Beispiel ein Bluetooth Modul. Das Funksignal des Bluetooth Standards ist in der Regel ausreichend, um ein bis zwei Wände zu durchdringen, sodass ein portables Telekommunikationsgerät 302, zum Beispiel ein Smartphone der Eltern, auch dann Warnsignale von dem tragbaren Gerät 200 empfangen kann, wenn sich die Eltern zum Beispiel kurzfristig in einem anderen Raum aber noch in räumlicher Nähe zum Kind befinden. Auf dem Telekommunikationsgerät 302 kann sich eine Software befinden, die auf Basis der von dem Armband 200 empfangenen Daten (insbesondere also Vorhersageergebnisse bezüglich physiologischer Zustände, optional aber auch Rohdaten oder aus den Rohdaten abgeleitete Vitalparameter) eine Ausgabe erzeugt. Beispielsweise kann die Ausgabe eine GUI umfassen, die über das Display des Smartphones angezeigt wird und zum Beispiel eine Warnung bezüglich des vorhergesagten physiologischen Zustands und/oder eine Handlungsempfehlung enthält. Die Empfehlung kann zum Beispiel darin bestehen, das Kind umzudrehen, ihm Nahrung zu geben, die Temperatur im Raum zu senken oder zu erhöhen oder dergleichen. Zusätzlich oder alternativ zu der optischen Ausgabe über das Display kann die Betreuungsperson auch akustisch gewarnt werden, zum Beispiel durch einen Alarmton oder durch das Aktivieren der Vibrier-Funktion des Smartphones.

**Figur 4** zeigt ein Blockdiagramm eines Bereichs eines tragbaren Geräts mit mehreren Sensoren. Das tragbare Gerät 200 beinhaltet eine Batterie 406 sowie ein oder mehrere Prozessoren 402, die zum Beispiel als Mikroprozessoren ausgebildet sein können.

Das Gerät enthält eine Schnittstelle 404 zum Datenaustausch mit dem portablen Telekommunikationsgerät 302, zum Beispiel eine Funkschnittstelle. Vorzugsweise enthält sie zudem eine Schnittstelle 403 zum Datenaustausch mit einem Server Computersystem. Die Schnittstelle 403 kann zum Beispiel eine Mobilfunkverbindung oder eine WLAN Verbindung sein, um mit dem Server Computersystem über das Internet Daten austauschen zu können.

Auf dem tragbaren Gerät ist eine Auswertungssoftware 408 installiert. Die Software kann ein oder mehrere prädiktive Modelle 410 enthalten, die zum Beispiel jeweils zur Vorhersage eines bestimmten physiologischen Zustands (zum Beispiel erhöhtes Risiko für den plötzlichen Kindstod, Auftreten eines Hungergefühls, Auftreten von Fieber, etc.) trainiert wurden. Anstatt der Modelle können aber auch regelbasierte Algorithmen verwendet werden.

Das Sensormodul 202 beinhaltet ein oder mehrere Sensoren 418 zur Erfassung von Vitalparametern. Insbesondere umfasst das Modul 202 einen PPG Sensor 412, aus dessen Rohdaten eine Vielzahl von relevanten Vitalparametern abgeleitet werden können, darunter zum Beispiel die Herzfrequenz, die Atemfrequenz, die Sauerstoffkonzentration des Blutes, die Glukosekonzentration des Blutes sowie einige weitere als Kontrolle bei der SIDS-Vorhersage dienende Vitalparameter bzw. Blutkomponenten. In manchen Ausführungsformen enthält das Gerät 200 weitere Sensoren für Vitalparameter wie zum Beispiel einen Sensor für die Hauttemperatur 414, ein Gyroskop 416 zur Erfassung von Bewegungen des Kindes und/oder ein Mikrofon 418.

Außerdem kann das Sensormodul 202 weitere Sensoren 422 zur Erfassung von Umgebungsparametern beinhalten, zum Beispiel eine Luftfeuchtigkeitssensor 424, einen Sensor 426 für die Umgebungstemperatur, und/oder einen Sensor für die tägliche oder stündliche UV-Strahlendosis 428. Beispielsweise kann der Sensor 428 dazu dienen, die UV-Lichtdosis, der das Kind im Laufe eines Tages ausgesetzt war, zu erfassen. Ist die empfohlene maximale Dose erreicht bzw. überschritten, kann die Auswertungssoftware eine Warnung an die App des Smartphones senden, dass das Kind vor weiterer Sonnenexposition geschützt werden muss. Die Aufzeichnung der täglichen UV-Lichtdosis über einige Zeit hinweg kann jedoch auch dabei helfen, eine Unterversorgung mit Sonnenlicht festzustellen.

Je nach Ausführungsform können verschiedene Sensoren verschiedener Hersteller verwendet werden, die sich teils in der Art und Weise der Verarbeitung der erfassten Messdaten unterscheiden. Beispielsweise geben Temperatursensoren üblicherweise die Temperatur in Grad Celsius oder Grad Fahrenheit an. Ein PPG-Sensorsignalen 112 liefert dagegen ein oder mehrere Lichtspektren, wobei erst durch eine nachträgliche Verarbeitung der Spektren ein oder mehrere Vitalparameter wie zum Beispiel die Sauerstoffkonzentration des Blutes oder die Glukosekonzentration erhalten werden.

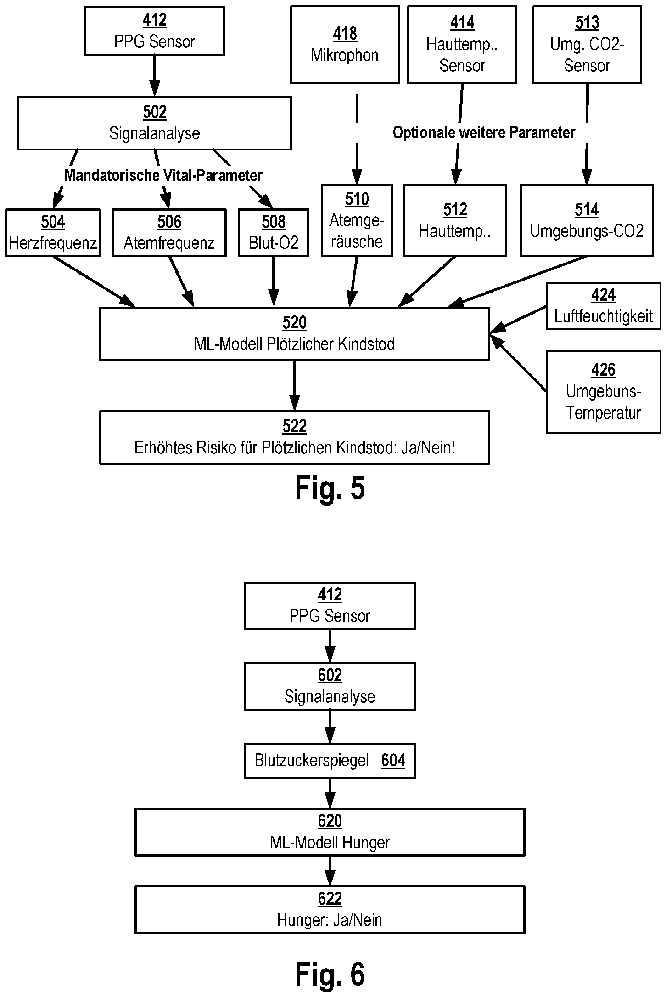

**Figur 5** zeigt ein Diagramm bezüglich der Ableitung und Verwendung verschiedener Parameter für die Vorhersage eines erhöhten SIDS-Risikos. Beispielsweise kann die Auswertungssoftware auf dem tragbaren Gerät 200 ein SIDS-Modell 520 beinhalten, welches zumindest die Herzfrequenz 504, die Atemfrequenz 506 und die Sauerstoffkonzentration im Blut des Kindes 508 als Eingabeparameter benötigt. Diese Vitalparameter können zum Beispiel durch eine Signalanalyse 502 auf Basis der Rohdaten bzw. Spektraldaten eines PPG-Sensors 412 erhalten werden. Die besagten drei Vitalparameter sind immer verfügbar, solange das Kind nur das tragbare Gerät am Körper trägt. Das SIDS-Modell 520 ist dazu ausgebildet, auch weitere Eingabeparameter auszuwerten, um die Vorhersage eines erhöhten Risikos für den plötzlichen Kindstod noch genauer zu machen. Hierzu gehören zum Beispiel einige Kontrollparameter in Form von Blut-Parameterwerten, die z.B. ebenfalls aus den Rohdaten des PPG Sensors abgeleitet werden können (hier nicht gezeigt). Außerdem können hierzu weitere Vitalparameter gehören wie zum Beispiel die von einem Temperatursensor 414 des Geräts erfasste Hauttemperatur 512, von einem Mikrofon 418 aufgezeichnete Atemgeräusche 510, von einem Kohlendioxidsensor 513 erfasste Kohlendioxidkonzentration in der Umgebungsluft, die Luftfeuchtigkeit 424 und/oder die Umgebungstemperatur 426. Eine Analyse der Atemgeräusche kann z.B. dazu dienen, zu ermitteln, ob die Atmung durch Obstruktion beeinflusst ist, was ein erhöhtes SIDS-Risiko implizieren würde. Auch Bewegungsdaten und/oder Videodaten (hier nicht gezeigt) können in die Vorhersage eingehen, da sie z.B. erkennen lassen, ob das Kind aktiv ist bzw. ob das Kind auf dem Bauch oder Rücken liegt.

Falls die Vorhersage ergibt, dass das Kind aktuell oder in Kürze ein erhöhtes Risiko für das Eintreten des plötzlichen Kindstodes hat, sendet das Gerät 200 eine Alarmnachricht 522 entweder direkt an das Smartphone der Betreuungsperson oder indirekt zunächst an den Servercomputer, wo das Vorhersageergebnis gegebenenfalls noch weiter verfeinert werden kann anhand von Daten, die externe Sensoren über die Basisstation zur Verfügung stellen. Das verfeinerte Vorhersageergebnis wird daraufhin, sofern auch das verfeinerte Vorhersageergebnis ein erhöhtes Risiko für das Auftreten des plötzlichen Kindstodes ergibt, von dem Server-Computersystem über das Netzwerk an das Smartphone der Betreuungsperson weitergeleitet und dort ausgegeben.

**Figur 6** zeigt ein Diagramm bezüglich der Ableitung und Verwendung eines Parameters für die Vorhersage von Hungergefühlen. Auch hier kann zunächst der PPG Sensor 412 zur Erfassung ein oder mehrerer Lichtspektren, die von der Haut des Kindes und den Gefäßen in der Haut reflektiert werden, zum Einsatz kommen. Aus den Rohdaten ermittelt die Auswertungssoftware per Signalanalyse 602 zumindest einen abgeleiteten Vitalparameter, nämlich den Blutzuckerspiegel 604. Zumindest der Blutzuckerspiegel und optional noch weitere Parameter gehen als Eingangsparameter in die Vorhersage eines aktuellen oder künftigen Hungergefühls durch ein trainiertes prädiktives "Hunger"-Modell 620 ein. Das Ergebnis 622 der Vorhersage, ob und gegebenenfalls wann ein Hungergefühl vorliegt bzw. vorliegen wird, wird entweder direkt per Nahfeldsignal an das Smartphone gesendet oder indirekt über das Servercomputer System, wobei der Server Computersystem insbesondere dazu dient, das Vorhersageergebnis durch Einbeziehung weiterer Daten weiterer Sensoren und/oder durch zusätzliche, ggf. rechenaufwändige Analysen, zu verfeinern.

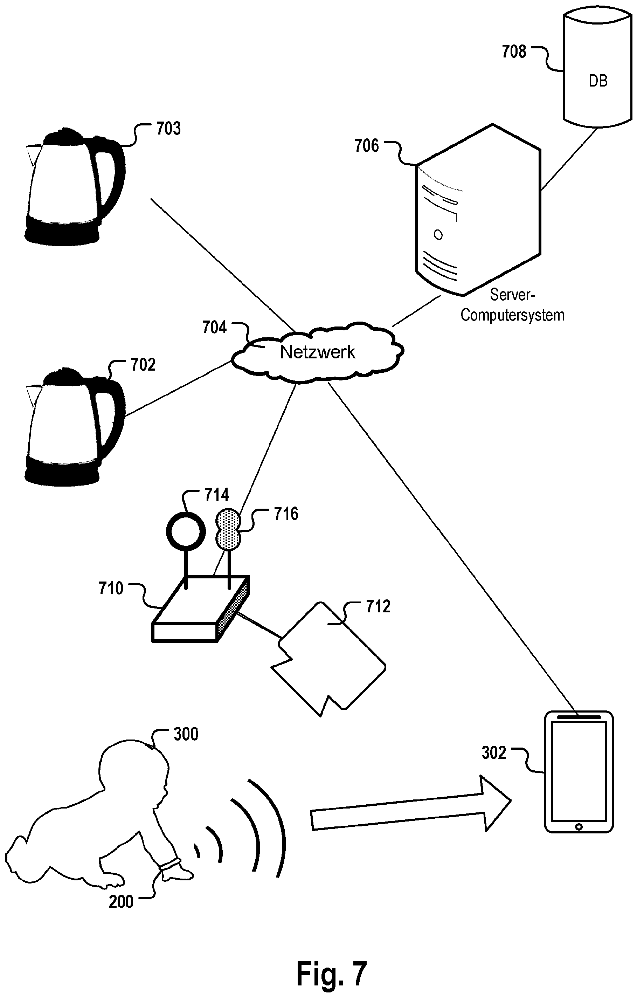

**Figur 7** zeigt ein System zur Überwachung der Gesundheit eines Kindes mit mehreren Komponenten. Das System umfasst zumindest das tragbare Gerät 200, das hier zum Beispiel als Armband zur Befestigung am Handgelenk eines Kindes 300 ausgebildet ist.

Das System kann außerdem ein oder mehrere portable Telekommunikationsgeräte 302, typischerweise Smartphones der Betreuungspersonen, beinhalten, auf welchem eine Software installiert ist, die mit der Auswertungssoftware des Geräts 200 interoperabel ist, um Daten mit dieser austauschen zu können. Beispielsweise können die Besitzer des Telekommunikationsgeräts 302 per Push Nachricht von der Auswertungssoftware über kritische physiologische Zustände des Kindes informiert werden und/oder per Pull Funktionalität Statusdaten oder historische Daten bezüglich der physiologischen Zustände des Kindes 300 aktiv von dem tragbaren Gerät 200 anfordern.

Das System kann außerdem ein Servercomputer-System 706 beinhalten, welches über ein Netzwerk 704, zum Beispiel das Internet, mit dem tragbaren Gerät 200 und der Auswertungssoftware 408 verbunden ist. Beispielsweise können die von dem Gerät 200 erfassten und gegebenenfalls abgeleiteten Daten sowie Vorhersageergebnisse je nach Dringlichkeit und Konfiguration sofort oder zum Beispiel während des Ladevorgangs der Batterie über das Netzwerk an das Server-Computersystem übermittelt werden. Das Server-Computersystem dient insbesondere dazu, die empfangenen Daten von ein oder mehreren Geräten 200 oder externen Sensoren 712 in einer Datenbank 708 zu speichern. Außerdem können auf dem Server-Computersystem Vorhersageergebnisse, die über das Netzwerk 704 von dem tragbaren Gerät 200 und dessen Sensoren empfangen werden, verfeinert und präzisiert werden. Dies kann insbesondere dadurch geschehen, dass zusätzliche Daten, die von externen Sensoren 712 ermittelt und direkt über das Netzwerk (z.B. Internet) oder indirekt über eine Basisstation 710 an das Server-Computersystem übermittelt wurden, zusätzlich berücksichtigt werden und/oder dadurch, dass der Server-Computersystem komplexe, rechenaufwendige Analysen vornimmt. Beispielsweise kann in oder an dem Bett, in welchem das Kind normalerweise schläft, ein Mikrofon 716 und/oder eine Kamera 712 (insbesondere Wärmebildkamera) oder sonstige weitere Sensoren 714 als externe Sensoren installiert sein. Diese externen Sensoren sind entweder direkt über das Netzwerk oder indirekt über eine Basisstation 710 kommunikativ an das Server-Computersystem 706 gekoppelt und können Daten an dieses senden. Beispielsweise kann eine Server-Applikation auf dem Server-Computersystem eine Bildanalyse der Videodaten der Kamera 712 durchführen, zum Beispiel um zu erkennen, ob das Kind sich in Rückenlage oder Bauchlage befindet, was ein wichtiger prognostischer Faktor für das Risiko des plötzlichen Kindstodes ist.

Gemäß einer Ausführungsform handelt es sich bei dem externen Sensor um eine Videokamera, insbesondere eine Wärmebild-Videokamera, welche über eine Schnittstelle zur Nahfeldkommunikation (z.B. Funk, insb. Bluetooth, oder WLAN) kommunikativ mit dem portablen Telekommunikationsgerät verbunden ist, um den Betreuungspersonen eine Überwachung des Babys per Videosignal zu ermöglichen. Vorzugsweise ist die Videokamera portabel und frei aufstellbar und ist z.B. per WLAN über das Internet kommunikativ an den Server-Computer koppelbar, vorzugsweise auch ohne eine Basisstation. Dies kann den Vorteil haben, dass die Eltern ohne größeren Installationsaufwand auch die Kamera in der Umgebung ihres Kindes installieren können, z.B. wenn sie auf Reisen sind, sodass die Mobilität der Eltern erhöht wird.

Erfindungsgemäß ist die Auswertungssoftware des Geräts 200 und optional auch die mit dieser Auswertesoftware interoperable Applikation auf dem Smartphone 302 operativ über das Netzwerk 704 gekoppelt an ein oder mehrere Geräte 702, 703, die der Vorbereitung oder Zubereitung von Nahrung für das Kind dienen. Bei den Geräten 702, 703 kann es sich zum Beispiel um eine Mikrowelle, eine Wasserkocher, ein Gerät zur Erwärmung von Milch oder Babynahrung etc. handeln. Falls das tragbare Gerät 200 mittels der Auswertungssoftware erkennt bzw. vorhersagt, dass das Kind aktuell oder in naher Zukunft ein Hungergefühl hat oder haben wird, kann die Auswertungssoftware automatisch einen Steuerbefehl an eines oder mehrere der Geräte 702, 703 senden, um dieses dazu veranlassen, die Nahrungsvorbereitung zu beginnen. Vorzugsweise wird der Steuerbefehl jedoch nicht direkt an die besagten Geräte 702, 703 gesendet, sondern zunächst an die Software auf dem Smartphone 302. In Antwort auf den Empfang des Steuerbefehls fordert die Smartphone-Software den Benutzer dazu auf, den Versand des Steuerbefehls an das betreffende Gerät freizugeben. Das Smartphone sendet sodann nach Erhalt der Freigabe durch den Benutzer den Steuerbefehl an das betroffene Gerät 702, 703. Hierdurch wird sichergestellt, dass die Auswertesoftware nicht ein Gerät automatisch aus der Ferne aktiviert, ohne dass die Betreuungspersonen davon wissen, da dies ein Sicherheitsrisiko darstellen könnte.

Bei dem Server-Computersystem 706 kann es sich um einen konventionellen, monolithischen Servercomputer handeln. Es kann sich aber auch um eine verteilte Serverarchitektur handeln, insbesondere um ein Cloud-Computersystem.

## Patentansprüche

1. Tragbares Gerät (200),
- wobei das tragbare Gerät zum Tragen am Körper eines Kindes (300) ausgebildet ist, wobei das Kind ein Baby oder Kleinkind ist;
- wobei das Gerät umfasst:
• einen oder mehrere Sensoren (412, 414, 416) zur Erfassung von mehreren Vitalparametern des Kindes, wobei die Vitalparameter zumindest umfassen die Herzfrequenz, die Sauerstoffsättigung, und die Atemfrequenz, wobei zumindest einer der Sensoren für die Vitalparameter dazu ausgebildet ist, die Blutzuckerkonzentration des Kindes in nicht-invasiver Weise zu messen; und
• eine Schnittstelle (403, 404) zur Übertragung eines Vorhersageergebnisses bezüglich zumindest eines aktuellen oder künftigen physiologischen Zustands an ein mobiles Telekommunikationsgerät (302) eines Nutzers und/oder an ein Server-Computersystem;
• eine Auswertungssoftware (408), die dazu konfiguriert ist, den zumindest einen aktuellen oder künftigen physiologischen Zustand des Kindes vorherzusagen als Funktion der von den Sensoren gemessenen Herzfrequenz, Sauerstoffsättigung, und Atemfrequenz, wobei der zumindest eine physiologische Zustand ein Zustand eines erhöhten Risikos des plötzlichen Kindstods ist, wobei die Auswertungssoftware dazu konfiguriert, ist, zumindest die Herzfrequenz, die Sauerstoffsättigung, und die Atemfrequenz als Input zu verwenden, um das Vorliegen eines erhöhten Risikos für den plötzlichen Kindstod vorherzusagen;
wobei die Auswertungssoftware dazu ausgebildet ist, einen weiteren physiologischen Zustand in Form eines aktuell oder künftig vorliegenden Hungergefühls des Kindes als Funktion zumindest der gemessenen Blutzuckerkonzentration vorherzusagen, und/oder einen künftigen Zeitprunkt des Auftretens des Hungergefühls vorherzusagen, wobei das Hungergefühl vorhergesagt wird, wenn der aktuelle oder künftige Blutzuckerspiegel unterhalb eines vordefinierten Grenzwertes liegt,
wobei die Auswertungssoftware direkt oder mittels einer Softwareapplikation des Telekommunikationsgeräts an ein elektronisches Gerät (702) zur Nahrungsvorbereitung kommunikativ gekoppelt ist, wobei die Auswertungssoftware und optional auch die Softwareapplikation des Telekommunikationsgeräts dazu konfiguriert ist, in Antwort auf die Vorhersage, dass bei dem Kind jetzt oder in der Zukunft ein Hungergefühl vorliegt, das elektronische Gerät zur Vorbereitung der Nahrung für das Kind zu veranlassen.

2. Das tragbare Gerät nach Anspruch 1, wobei die Auswertungssoftware dazu konfiguriert ist, das Vorliegen eines erhöhten Risikos für den plötzlichen Kindstod als Funktion von ein oder mehreren weiteren Parametern vorherzusagen, wobei die ein oder mehreren weiteren Parameter umfassen:
- die Hauttemperatur des Kindes;
- die Umgebungstemperatur; und/oder
- die Umgebungsluftfeuchte.

3. Das Gerät nach einem der vorigen Ansprüche,
- wobei die Sensoren einen photoplethysmograpischen Sensor, hier als PPG Sensor (412) bezeichnet, umfassen,
- wobei die Auswertungssoftware dazu ausgebildet ist, aus den vom PPG Sensor erfassten Signalen sowohl die Herzfrequenz, die Sauerstoffsättigung, als auch die Atemfrequenz des Kindes abzuleiten und als Input der Auswertungssoftware zur Verfügung zu stellen.

4. Das Gerät nach einem der vorigen Ansprüche,
- wobei die einen oder mehreren Sensoren einen Sensor zur Erfassung zumindest eines Blutparameters des Kindes umfasst, wobei der zumindest eine Blutparameter insbesondere eine Methämoglobin-Konzentration und/oder eine Carboxyhämoglobin-Konzentration und/oder eine CO₂ Konzentration im Blut des Kindes ist, wobei der Sensor zur Erfassung des Blutparameters insbesondere als der PPG-Sensor gemäß Anspruch 3 ausgebildet sein kann;
- wobei die Auswertungssoftware dazu ausgebildet ist, den zumindest einen Blutparameter als zusätzlichen Inputparameter zu verwenden, um die Falsch-Positivrate der Vorhersage des erhöhten Risikos des plötzlichen Kindstods durch die Auswertungssoftware zu reduzieren.

5. Das Gerät nach einem der vorigen Ansprüche,
- wobei das Gerät mindestens einen Sensor zur Ermittlung mindestens eines weiteren Vitalparameters und/oder Umgebungsparameters umfasst oder eine Schnittstelle zum Empfang des weiteren Vitalparameters und/oder Umgebungsparameters von einem externen Sensor, wobei der zumindest eine weitere Umgebungsparameter ausgewählt ist aus einer Gruppe umfassend:
• die CO₂-Konzentration der Umgebungsluft,
• Videodaten des Kindes, insbesondere Videodaten einer Infrarot-Kamera;
• Von einem Mikrofon erfasste akustische Daten; und
• Bewegungsdaten, welche die Bewegungsaktivität des Kindes charakterisieren;
- wobei die Auswertungssoftware dazu ausgebildet ist, den zumindest einen weiteren Vitalparameter und/oder Umgebungsparameter zusätzlichen Inputparameter zu verwenden, um das Vorliegen eines erhöhten Risikos des plötzlichen Kindstods vorherzusagen.

6. Das Gerät nach einem der vorigen Ansprüche,
- wobei die Sensoren einen photoplethysmograpischen Sensor, hier als PPG Sensor (412) bezeichnet, umfassen,
- wobei die Auswertungssoftware dazu ausgebildet ist, aus den vom PPG Sensor erfassten Signalen neben der Herzfrequenz, der Sauerstoffsättigung, und der Atemfrequenz des Kindes auch die Blutzuckerkonzentration des Kindes abzuleiten und zumindest die Blutzuckerkonzentration als Input zumindest zur Vorhersage des Hungergefühls zur Verfügung zu stellen.

7. Das Gerät nach einem der vorigen Ansprüche,
- wobei die Auswertungssoftware dazu ausgebildet ist, das aktuelle oder künftige Vorliegen eines physiologisch problematischen Zustands des Kindes zu erkennen,
• wenn ein Wert zumindest eines Vitalparameter außerhalb eines vorab festgelegten Normbereichs liegt; und/oder
• wenn ein Muster der Werte mehrerer Vitalparameter erkannt wird, welches einen aktuellen oder künftigen problematischen physiologischen Zustand des Kindes anzeigt, wobei das Muster auch erkannt werden kann, wenn alle Vitalparameter einzeln jeweils innerhalb ihres Normbereichs liegen; und
- wobei die Auswertungssoftware dazu ausgebildet ist, in Reaktion auf das Erkennen des aktuell oder künftig vorliegenden physiologisch problematischen Zustands eine Nachricht bezüglich des vorhergesagten problematischen physiologischen Zustands an das mobile Telekommunikationsgerät (302) und/oder das Server-Computersystem zu senden.

8. Das Gerät nach einem der vorigen Ansprüche,
- wobei die Auswertungssoftware dazu ausgebildet ist, selektiv das aktuelle oder künftige Vorliegen eines physiologisch problematischen Zustands des Kindes zu erkennen, der sofortige Intervention erfordert, wobei dieser physiologisch problematische Zustand das erhöhte Risiko für den plötzlichen Kindstod umfasst; und
- zumindest einige der vom tragbaren Gerät gemessenen oder abgeleiteten Vitalparameter oder Vorhersagezwischenergebnisse über ein Netzwerk an das Server-Computersystem weiterzuleiten, um diesem die Vorhersage physiologischer Zustände, die keine sofortige Intervention erfordern, und/oder eine Berechnung einer verfeinerten finalen Vorhersage zu ermöglichen.

9. Das Gerät nach einem der vorigen Ansprüche, wobei es sich bei dem Gerät (200) um ein Armband oder Band am Fußgelenk oder Bein handelt.

10. Das Gerät nach Anspruch 9,
- wobei die Sensoren einen oder mehrere Drucksensoren umfassen, die dazu ausgebildet sind, den Anpressdruck des Gerätes am Körper des Kindes zu erfassen,
- wobei die Auswertungssoftware dazu ausgebildet ist, auf Basis des gemessenen Anpressdrucks zu erkennen, ob der Anpressdruck innerhalb eines vordefinierten zulässigen Anpressruckbereichs ist, innerhalb welchem die ein oder mehreren Sensoren zur Erfassung der Vitalwerte korrekt arbeiten können,
- wobei die Auswertungssoftware dazu ausgebildet ist, eine Warnung über ein Signalelement des Geräts (200) an den Nutzer und/oder über die Schnittstelle an das Telekommunikationsgerät auszugeben, falls der gemessene Anpressruck außerhalb des zulässigen Anpressdruckbereichs ist; und/oder
- wobei die Auswertungssoftware dazu ausgebildet ist, eine Messung von Vitalparametern durch die ein oder mehreren Sensoren so lange zu unterbinden, bis der Anpressdruck wieder innerhalb des zulässigen Anpressruckbereichs ist.

11. Das Gerät nach einem der vorigen Ansprüche, wobei das Gerät dazu konfiguriert ist,
- nur dann, falls die Auswertungssoftware das aktuelle oder künftige Vorliegen eines physiologisch problematischen Zustands, insbesondere ein erhöhtes Risiko für einen plötzlichen Kindstod und/oder ein Hungergefühl, oder das Vorliegen eines Vital- oder Umweltparameters in einem gesundheitskritischen Wertebereich festgestellt hat, eine Nachricht an das Nutzergerät zu senden; und
- andernfalls die erfassten Vitalparameter sowie optional erfasste Umgebungsparameter automatisch zu speichern ohne eine Nachricht zu senden.

12. Das Gerät nach einem der vorigen Ansprüche,
- wobei die Schnittstelle (404) zur Übertragung von Daten an das Telekommunikationsgerät eine Schnittstelle zur Datenübertragung über ein Nahfeldsignal, insbesondere über ein Funksignal, insbesondere eine Bluetooth-Schnittstelle oder ZigBee-Schnittstelle, ist,
- wobei das tragbare Gerät in einem strahlungsarmen und in einem strahlungsnormalen Betriebszustand betreibbar ist;
- wobei das tragbare Gerät dazu konfiguriert ist, im normalen Betriebsmodus, wenn kein physiologischer Zustand vorhergesagt und kein Vital- oder Umgebungsparameter gemessen wird, der eine sofortige Intervention erfordert, im strahlungsarmen Betriebszustand zu arbeiten; und wobei das tragbare Gerät dazu konfiguriert, falls die Auswertungssoftware das aktuelle oder künftige Vorliegen eines physiologisch problematischen Zustands, insbesondere ein erhöhtes Risiko für einen plötzlichen Kindstod und/oder ein Hungergefühl, oder das Vorliegen eines Vital- oder Umweltparameters in einem gesundheitskritischen Wertebereich festgestellt hat, automatisch in den strahlungsnormalen Betriebsmodus zu wechseln.

13. Das tragbare Gerät nach einem der vorigen Ansprüche,
- wobei das tragbare Gerät ein oder mehrere Umweltparameter-Sensoren umfasst ausgewählt aus einer Gruppe beinhaltend:
• ein Thermometer, das zum Messen der Umgebungstemperatur;
• ein Messgerät zum Messen der Umgebungsluftfeuchte;
• Gase, insbesondere CO₂;
• UV-Sensor zur Erfassung einer kumulativen UV-Strahlendosis, insbesondere einer täglichen kumulativen UV-Strahlendosis;
- und/oder wobei die Sensoren des tragbaren Geräts zur Erfassung der Vitalparameter weitere Sensoren umfassen ausgewählt aus einer Gruppe beinhaltend:
• Beschleunigungssensor zum Erfassen der Lage (Rückenlage, Bauchlage) des Kindes;
• Temperatursensor zum Erfassen der Hauttemperatur;
• ein Mikrophon zum Erfassen von Umgebungsgeräuschen und/oder von Geräuschen des Kindes;
• Videokamera, insbesondere eine Wärmebildkamera.

14. Das tragbare Gerät nach einem der vorigen Ansprüche, wobei die Vorhersagesoftware zumindest ein prädiktives Modell zur Vorhersage des zumindest einen physiologischen Zustands beinhaltet, wobei das zumindest eine prädiktive Modell ein durch ein maschinelles Lernverfahren auf Basis eines Trainingsdatensatzes erzeugtes Modell ist.

15. System beinhaltend das tragbare Gerät nach einem der vorigen Ansprüche und ein oder mehrere der folgenden weiteren Komponenten:
- das portable Telekommunikationsgerät, wobei auf dem portablen Telekommunikationsgerät eine Nutzersoftware instanziiert ist, wobei die Nutzersoftware mit der Auswertungssoftware interoperabel ist und dazu ausgebildet ist, die von dem tragbaren Gerät über die Schnittstelle empfangenen Vorhersageergebnisse dem Nutzer anzuzeigen und/oder dem Nutzer die Konfiguration der Auswertungssoftware zu ermöglichen; und/oder
- das Server-Computersystem; und/oder
- eine Basisstation, an welche ein oder mehrere externe Sensoren zur Messung von Vitalparametern des Kindes oder von Umgebungsparametern der Umgebung des Kindes gekoppelt sind; und/oder
- ein oder mehrere der externen Sensoren, insbesondere eine Videokamera, insbesondere eine Wärmebildvideokamera.

16. Verfahren zur Bereitstellung eines tragbaren Geräts (200) zur Überwachung des physiologischen Zustands eines Kindes (300), umfassend:
- Bereitstellen (102) eines Trainingsdatensatzes beinhaltend mehrere Datensätze, wobei in jedem Datensatz zumindest ein physiologischer Zustand des Kindes verknüpft gespeichert ist mit Vitalparametern des Kindes, wobei die Vitalparameter zumindest umfassen die Herzfrequenz, die Sauerstoffsättigung, und die Atemfrequenz;
- Durchführen (104) eines maschinellen Lernverfahrens auf den Trainingsdaten zur Erzeugung zumindest einen prädiktiven Modells (520, 620), wobei das Modell zur Vorhersage des physiologischen Zustands des Kindes auf Basis zumindest der Herzfrequenz, der Sauerstoffsättigung, und der Atemfrequenz und ausgebildet ist;
- Installation (106) einer Auswertungssoftware (408), die das zumindest eine prädiktive Modell (410, 520, 620) beinhaltet auf dem tragbaren Gerät, wobei das Gerät zum Tragen am Körper eines Kindes (300) ausgebildet ist, wobei das Kind ein Baby oder Kleinkind ist, wobei das Gerät umfasst:
• einen oder mehrere Sensoren (412, 414, 416) zur Erfassung von mehreren Vitalparametern des Kindes, wobei die Vitalparameter zumindest umfassen die Herzfrequenz, die Sauerstoffsättigung, und die Atemfrequenz, wobei die Auswertungssoftware dazu ausgebildet ist, das zumindest eine prädiktive Modell zur Vorhersage des physiologischen Zustands auf Basis der von den Sensoren erfassten Herzfrequenz, die Sauerstoffsättigung, und die Atemfrequenz zu verwenden, wobei zumindest einer der Sensoren für die Vitalparameter dazu ausgebildet ist, die Blutzuckerkonzentration des Kindes in nicht-invasiver Weise zu messen; und
• eine Schnittstelle (403, 404) zur Übertragung eines Vorhersageergebnisses bezüglich des zumindest einen physiologischen Zustands an ein mobiles Telekommunikationsgerät (302) eines Nutzers und/oder an ein Server-Computersystem, wobei der zumindest eine physiologische Zustand ein Zustand eines erhöhten Risikos des plötzlichen Kindstods ist, wobei die Auswertungssoftware dazu konfiguriert, ist, zumindest die Herzfrequenz, die Sauerstoffsättigung, und die Atemfrequenz als Input zu verwenden, um das Vorliegen eines erhöhten Risikos für den plötzlichen Kindstod vorherzusagen,
wobei die Auswertungssoftware dazu ausgebildet ist, einen weiteren physiologischen Zustand in Form eines aktuell oder künftig vorliegenden Hungergefühls des Kindes als Funktion zumindest der gemessenen Blutzuckerkonzentration vorherzusagen, und/oder einen künftigen Zeitprunkt des Auftretens des Hungergefühls vorherzusagen, wobei das Hungergefühl vorhergesagt wird, wenn der aktuelle oder künftige Blutzuckerspiegel unterhalb eines vordefinierten Grenzwertes liegt,
• wobei die Auswertungssoftware direkt oder mittels einer Softwareapplikation des Telekommunikationsgeräts an ein elektronisches Gerät (702) zur Nahrungsvorbereitung kommunikativ gekoppelt ist, wobei die Auswertungssoftware und optional auch die Softwareapplikation des Telekommunikationsgeräts dazu konfiguriert ist, in Antwort auf die Vorhersage, dass bei dem Kind jetzt oder in der Zukunft ein Hungergefühl vorliegt, das elektronische Gerät zur Vorbereitung der Nahrung für das Kind zu veranlassen.

## Claims

1. A wearable device (200),
- wherein the wearable device is configured to be worn on the body of a child (300), wherein the child is a baby or toddler;
- wherein the device comprises:
• one or more sensors (412, 414, 416) for sensing a plurality of vital parameters of the child, wherein the vital parameters comprise at least heart rate, oxygen saturation, and respiratory rat, wherein at least one of the sensors for the vital parameters is configured to measure the blood sugar concentration of the child non-invasively; and
• an interface (403, 404) for transmitting a prediction result relating to at least a current or future physiological state to a mobile telecommunication device (302) of a user and/or to a server-computer system;
• evaluation software (408) configured to predict at least a current or future physiological state of the child as a function of the heart rate, oxygen saturation, and respiratory rate measured by the sensors, wherein the at least one physiological state is a state of an increased risk of sudden infant death syndrome, wherein the evaluation software is configured to use at least the heart rate, oxygen saturation and respiratory rate as input to predict the presence of an increased risk of sudden infant death syndrome;
wherein the evaluation software is configured to predict a further physiological state in the form of a current or future feeling of hunger in the child as a function of at least the measured blood sugar concentration, and/or to predict a future time of occurrence of the feeling of hunger, wherein the feeling of hunger is predicted when the current or future blood sugar level is below a predefined limit value,
wherein the evaluation software is communicatively coupled to an electronic food preparation appliance (702) directly or by means of a software application of the telecommunication device, wherein the evaluation software and optionally also the software application of the telecommunication device is configured to cause the electronic appliance to prepare food for the child in response to the prediction that the child is feeling hungry now or will do in the future.

2. The wearable device according to claim 1, wherein the evaluation software is configured to predict the presence of an increased risk of sudden infant death syndrome as a function of one or more further parameters, wherein the one or more further parameters comprise:
- the child's skin temperature;
- the ambient temperature; and/or
- the ambient air moisture.

3. The device according to any one of the preceding claims,
- wherein the sensors comprise a photoplethysmographic sensor, referred to here as a PPG sensor,
- wherein the evaluation software is configured to derive the heart rate, oxygen saturation and respiratory rate of the child from the signals detected by the PPG sensor and to make them available as input to the evaluation software.

4. The device according to any one of the preceding claims,
- wherein the one or more sensors comprises a sensor for detecting at least one blood parameter of the child, wherein the at least one blood parameter is in particular a methaemoglobin concentration and/or a carboxyhaemoglobin concentration and/or a CO₂ concentration in the blood of the child, wherein the sensor for detecting the blood parameter may be configured in particular as the PPG sensor according to claim 3;
- wherein the evaluation software is configured to use the at least one blood parameter as an additional input parameter in order to reduce the false positive rate of the prediction of the increased risk of sudden infant death syndrome by the evaluation software.

5. The device according to any one of the preceding claims,
- wherein the device comprises at least one sensor for determining at least one further vital parameter and/or environmental parameter, or an interface for receiving the further vital parameter and/or environmental parameter from an external sensor, wherein the at least one further environmental parameter is selected from a group comprising:
• the CO₂ concentration of the ambient air,
• video data of the child, in particular video data of an infrared camera;
• acoustic data detected by a microphone; and
• movement data characterising the child's movement activity;
- wherein the evaluation software is configured to use the at least one further vital parameter and/or environmental parameter as an additional input parameter in order to predict the presence of an increased risk of sudden infant death syndrome.

6. The device according to any one of the preceding claims,
- wherein the sensors comprise a photoplethysmographic sensor, referred to here as a PPG sensor,
- wherein the evaluation software is configured to derive the child's blood sugar concentration from the signals detected by the PPG sensor in addition to the heart rate, the oxygen saturation and the respiratory rate of the child and to provide at least the blood sugar concentration as input at least for predicting the feeling of hunger.

7. The device according to any one of the preceding claims,
- wherein the evaluation software is configured to recognise the current or future presence of a physiologically problematic state of the child,
• when a value of at least one vital parameter is outside a predetermined normal range; and/or
• when a pattern of values of multiple vital parameters is detected that indicates a current or future problematic physiological state of the child, wherein the pattern may be recognised even if all vital parameters are individually within their normal range; and
- wherein the evaluation software is configured to send a message regarding the predicted problematic physiological state to the mobile telecommunication device (302) and/or the server-computer system in response to the recognition of the current or future physiologically problematic state.

8. The device according to any one of the preceding claims,
- wherein the evaluation software is configured to selectively recognise the current or future presence of a physiologically problematic state of the child requiring immediate intervention, wherein this physiologically problematic state comprises in particular the increased risk of sudden infant death syndrome; and
- forwarding at least some of the vital parameters or intermediate prediction results measured or derived by the wearable device to the server-computer system via a network to enable the server-computer system to predict physiological states that do not require immediate intervention and/or calculate a refined final prediction.

9. The device according to any one of the preceding claims, wherein the device (200) is a bracelet or ankle strap or leg strap.

10. The device according to claim 9,
- wherein the sensors comprise one or more pressure sensors which are configured to detect the contact pressure of the device on the child's body,
- wherein the evaluation software is configured to recognise, on the basis of the measured contact pressure, whether the contact pressure is within a predefined permissible contact pressure range within which the one or more sensors for detecting the vital values are able to operate correctly,
- wherein the evaluation software is configured to issue a warning via a signalling element of the device (200) to the user and/or via the interface to the telecommunication device if the measured contact pressure is outside the permissible contact pressure range; and/or
- wherein the evaluation software is configured to prevent the measurement of vital parameters by the one or more sensors until the contact pressure is again within the permissible contact pressure range.

11. The device according to any one of the preceding claims, wherein the device is configured
- to only send a message to the user device if the evaluation software has detected the current or future presence of a physiologically problematic state, in particular an increased risk of sudden infant death syndrome and/or a feeling of hunger, or the presence of a vital or environmental parameter in a health-critical value range; and
- otherwise to automatically save the detected vital parameters and optionally detected environmental parameters without sending a message.

12. The device according to any one of the preceding claims,
- wherein the interface (404) for transmitting data to the telecommunication device is an interface for data transmission via a near-field signal, in particular via a radio signal, in particular a Bluetooth interface or ZigBee interface,
- wherein the wearable device is operable in a low-radiation operating mode and in a normal-radiation operating mode;
- wherein the wearable device, in the normal operating mode, is configured to operate in the low-radiation operating state when no physiological state is predicted and no vital or environmental parameter requiring immediate intervention is measured; and wherein the wearable device is configured to automatically switch to the radiation-normal operating mode if the evaluation software has detected the current or future presence of a physiologically problematic state, in particular an increased risk of sudden infant death syndrome and/or a feeling of hunger, or the presence of a vital or environmental parameter in a health-critical value range.

13. The wearable device according to any one of the preceding claims,
- wherein the wearable device comprises one or more environmental parameter sensors selected from a group including:
• a thermometer that measures the ambient temperature;
• a measuring device for measuring the ambient air moisture;
• gases, especially CO₂;
• UV sensor for detecting a cumulative UV radiation dose, in particular a daily cumulative UV radiation dose;
- and/or wherein the sensors of the wearable device for detecting the vital parameters comprise further sensors selected from a group comprising:
• acceleration sensor for detecting the position of the child (supine position, prone position);
• temperature sensor for detecting the skin temperature;
• a microphone for detecting ambient noises and/or noises made by the child;
• video camera, in particular a thermal imaging camera.

14. The wearable device according to any one of the preceding claims, wherein the prediction software comprises at least one predictive model for predicting the at least one physiological state, wherein the at least one predictive model is a model generated by a machine learning method on the basis of a training dataset.

15. A system comprising the wearable device according to any one of the preceding claims and one or more of the following further components:
- the portable telecommunication device, wherein a user software is set up on the portable telecommunication device, wherein the user software is interoperable with the evaluation software and is configured to display the prediction results received from the wearable device via the interface to the user and/or to enable the user to configure the evaluation software; and/or
- the server-computer system; and/or
- a base station to which one or more external sensors are coupled for measuring vital parameters of the child or environmental parameters of the child's surroundings; and/or
- one or more of the external sensors, in particular a video camera, in particular a thermal imaging video camera.

16. A method for providing a wearable device (200) for monitoring the physiological state of a child (300), comprising:
- providing (102) a training dataset comprising a plurality of datasets, wherein in each dataset at least one physiological state of the child is stored linked to vital parameters of the child, wherein the vital parameters comprise at least the heart rate, oxygen saturation, and respiratory rate;
- performing (104) a machine learning process on the training data to generate at least one predictive model (520, 620), wherein the model is configured to predict the physiological state of the child on the basis of at least the heart rate, oxygen saturation, and respiratory rate;
- installing (106) evaluation software (408) including the at least one predictive model (410, 520, 620) on the wearable device, wherein the device is configured to be worn on the body of a child (300), wherein the child is a baby or toddler, wherein the device comprises:
• one or more sensors (412, 414, 416) for detecting a plurality of vital parameters of the child, wherein the vital parameters comprise at least the heart rate, oxygen saturation, and respiratory rate, wherein the evaluation software is configured to use the at least one predictive model to predict the physiological state on the basis of the heart rate, oxygen saturation, and respiratory rate detected by the sensors, wherein at least one of the sensors for the vital parameters is configured to measure the child's blood sugar concentration non-invasively; and
• an interface (403, 404) for transmitting a prediction result relating to the at least one physiological state to a mobile telecommunication device (302) of a user and/or to a server-computer system, wherein the at least one physiological state is a state of an increased risk of sudden infant death syndrome, wherein the evaluation software is configured to use at least the heart rate, oxygen saturation and respiratory rate as input to predict the presence of an increased risk of sudden infant death syndrome,
wherein the evaluation software is configured to predict a further physiological state in the form of a current or future feeling of hunger in the child as a function of at least the measured blood sugar concentration, and/or to predict a future time of occurrence of the feeling of hunger, wherein the feeling of hunger is predicted when the current or future blood sugar level is below a predefined limit value,
• wherein the evaluation software is communicatively coupled to an electronic food preparation appliance (702) directly or by means of a software application of the telecommunication device, wherein the evaluation software and optionally also the software application of the telecommunication device is configured to cause the electronic appliance to prepare food for the child in response to the prediction that the child is feeling hungry now or will do in the future.

## Revendications

1. Dispositif vestimentaire (200),
- dans lequel le dispositif vestimentaire est configuré pour être porté sur le corps d'un enfant (300), dans lequel l'enfant est un bébé ou un tout-petit ;
- dans lequel le dispositif comprend :
• un ou plusieurs capteurs (412, 414, 416) destinés à détecter une pluralité de paramètres vitaux de l'enfant, dans lequel les paramètres vitaux comprennent au moins le rythme cardiaque, la saturation en oxygène et la fréquence respiratoire, dans lequel au moins l'un des capteurs de paramètres vitaux est configuré pour mesurer la glycémie sanguine de l'enfant de manière non invasive ; et
• une interface (403, 404) destinée à transmettre un résultat de prévision lié à au moins un état physiologique actuel ou futur à un dispositif de télécommunication mobile (302)d'un utilisateur et/ou à un système serveur-ordinateur ;
• un logiciel d'évaluation (408) configuré pour prévoir au moins un état physiologique actuel ou futur de l'enfant en fonction du rythme cardiaque, de la saturation en oxygène et de la fréquence respiratoire mesurés par les capteurs, dans lequel l'au moins état physiologique est un état de risque supérieur de syndrome de mort subite du nourrisson, dans lequel le logiciel d'évaluation est configuré pour utiliser au moins le rythme cardiaque, la saturation en oxygène et la fréquence respiratoire en entrée pour prévoir la présence d'un risque supérieur de syndrome de mort subite du nourrisson ;
dans lequel le logiciel d'évaluation est configuré pour prévoir un état physiologique supplémentaire sous la forme d'un sentiment de faim actuel ou futur chez l'enfant en fonction d'au moins la glycémie sanguine mesurée, et/ou pour prévoir un temps d'apparition futur du sentiment de faim, dans lequel le sentiment de faim est prévu lorsque la glycémie sanguine actuelle ou future est inférieure à une valeur limite prédéfinie,
dans lequel le logiciel d'évaluation est couplé en communication à un appareil de préparation de nourriture électronique (702) directement ou au moyen d'une application logicielle du dispositif de télécommunication, dans lequel le logiciel d'évaluation et éventuellement également l'application logicielle du dispositif de télécommunications sont configurés pour amener l'appareil électronique à préparer de la nourriture pour l'enfant en réponse à la prévision selon laquelle l'enfant éprouve de la faim maintenant ou l'éprouvera à l'avenir.

2. Dispositif vestimentaire selon la revendication 1, dans lequel le logiciel d'évaluation est configuré pour prévoir la présence d'un risque supérieur de syndrome de mort subite du nourrisson en fonction d'un ou de plusieurs paramètres supplémentaires, dans lequel le ou les paramètres supplémentaires comprennent :
- la température cutanée de l'enfant ;
- la température ambiante ; et/ou
- l'humidité de l'air ambiant.

3. Dispositif selon l'une quelconque des revendications précédentes,
- dans lequel les capteurs comprennent un capteur photopléthysmographique, appelé ici capteur PPG,
- dans lequel le logiciel d'évaluation est configuré pour dériver le rythme cardiaque, la saturation en oxygène et la fréquence respiratoire de l'enfant des signaux détectés par le capteur PPG et pour les rendre disponibles en entrée du logiciel d'évaluation.

4. Dispositif selon l'une quelconque des revendications précédentes,
- dans lequel le ou les capteurs comprennent un capteur destiné à détecter au moins un paramètre sanguin de l'enfant, dans lequel l'au moins un paramètre sanguin est en particulier une concentration en méthémoglobine et/ou une concentration en carboxyhémoglobine et/ou une concentration en CO₂ dans le sang de l'enfant, dans lequel le capteur destiné à détecter le paramètre sanguin peut être configuré en particulier en tant que capteur PPG selon la revendication 3 ;
- dans lequel le logiciel d'évaluation est configuré pour utiliser l'au moins un paramètre sanguin en tant que paramètre d'entrée supplémentaire afin de réduire le taux de faux positifs de la prévision du risque supérieur de syndrome de mort subite du nourrisson par le logiciel d'évaluation.

5. Dispositif selon l'une quelconque des revendications précédentes,
- dans lequel le dispositif comprend au moins un capteur destiné à déterminer au moins un paramètre vital et/ou paramètre environnemental supplémentaire, ou une interface destinée à recevoir le paramètre vital et/ou paramètre environnemental supplémentaire depuis un capteur externe, dans lequel l'au moins un paramètre environnemental supplémentaire est choisi dans un groupe comprenant :
• la concentration en CO₂ de l'air ambiant,
• des données vidéo de l'enfant, en particulier des données vidéos d'une caméra infrarouge ;
• des données acoustiques détectées par un microphone ; et
• des données de mouvement caractérisant l'activité de mouvement de l'enfant ;
- dans lequel le logiciel d'évaluation est configuré pour utiliser l'au moins un paramètre vital et/ou paramètre environnemental supplémentaire en tant que paramètre d'entrée supplémentaire afin de prévoir la présence d'un risque supérieur de syndrome de mort subite du nourrisson.

6. Dispositif selon l'une quelconque des revendications précédentes,
- dans lequel les capteurs comprennent un capteur photopléthysmographique, appelé ici capteur PPG,
- dans lequel le logiciel d'évaluation est configuré pour dériver la glycémie sanguine de l'enfant des signaux détectés par le capteur PPG en plus du rythme cardiaque, de la saturation en oxygène et de la fréquence respiratoire de l'enfant et pour fournir au moins la glycémie sanguine en entrée au moins pour prévoir le sentiment de faim.

7. Dispositif selon l'une quelconque des revendications précédentes,
- dans lequel le logiciel d'évaluation est configuré pour reconnaître la présence actuelle ou future d'un état physiologique problématique de l'enfant,
• lorsqu'une valeur d'au moins un paramètre vital est hors d'une plage normale prédéterminée ; et/ou
• lorsqu'une tendance de valeurs de plusieurs paramètres vitaux est détectée indiquant un état physiologique problématique actuel ou futur de l'enfant, dans lequel la tendance peut être reconnue même si tous les paramètres vitaux sont individuellement compris dans leur plage normale ; et
- dans lequel le logiciel d'évaluation est configuré pour envoyer un message concernant l'état physiologique problématique prévu au dispositif de télécommunication mobile (302) et/ou au système serveur-ordinateur en réponse à la reconnaissance de l'état physiologique problématique actuel ou futur.

8. Dispositif selon l'une quelconque des revendications précédentes,
- dans lequel le logiciel d'évaluation est configuré pour reconnaître sélectivement la présence actuelle ou future d'un état physiologique problématique de l'enfant exigeant une intervention immédiate, dans lequel cet état physiologique problématique comprend en particulier le risque supérieur de syndrome de mort subite du nourrisson ; et
- transférer au moins certains des paramètres vitaux ou des résultats de prévision intermédiaires mesurés ou dérivés par le dispositif vestimentaire au système serveur-ordinateur par l'intermédiaire d'un réseau pour permettre au système serveur-ordinateur de prévoir des états physiologiques ne requérant pas d'intervention immédiate et/ou calculer une prévision finale affinée.

9. Dispositif selon l'une quelconque des revendications précédentes, dans lequel le dispositif (200) est un bracelet, un ruban de cheville ou un ruban de jambe.

10. Dispositif selon la revendication 9,
- dans lequel les capteurs comprennent un ou plusieurs capteurs de pression qui sont configurés pour détecter la pression de contact du dispositif sur le corps de l'enfant,
- dans lequel le logiciel d'évaluation est configuré pour reconnaître, sur la base de la pression de contact mesurée, si la pression de contact est comprise dans une plage de pression de contact admissible prédéfinie au sein de laquelle le ou les capteurs de détection de valeurs vitales peuvent fonctionner correctement,
- dans lequel le logiciel d'évaluation est configuré pour émettre un avertissement par l'intermédiaire d'un élément de signalisation du dispositif (200) à l'utilisateur et/ou par l'intermédiaire de l'interface au dispositif de télécommunication si la pression de contact mesuré est hors de la plage de pression de contact admissible ; et/ou
- dans lequel le logiciel d'évaluation est configuré pour empêcher la mesure de paramètres vitaux par le ou les capteurs jusqu'à ce que la pression de contact soit à nouveau à l'intérieur de la plage de pression de contact admissible.

11. Dispositif selon l'une quelconque des revendications précédentes, dans lequel le dispositif est configuré
- pour uniquement envoyer un message au dispositif utilisateur si le logiciel d'évaluation a détecté la présence actuelle ou future d'un état physiologique problématique, en particulier un risque supérieur de syndrome de mort subite du nourrisson et/ou un sentiment de faim, ou la présence d'un paramètre vital ou environnemental dans une plage de valeurs critiques pour la santé ; et
- dans le cas contraire pour enregistrer automatiquement les paramètres vitaux détectés et éventuellement les paramètres environnementaux détectés sans envoyer de message.

12. Dispositif selon l'une quelconque des revendications précédentes,
- dans lequel l'interface (404) destinée à transmettre les données au dispositif de télécommunication est une interface de transmission des données par l'intermédiaire d'un signal de champ proche, en particulier par l'intermédiaire d'un signal radio, en particulier une interface Bluetooth ou une interface ZigBee,
- dans lequel le dispositif vestimentaire peut fonctionner en mode de fonctionnement à rayonnement faible et en mode de fonctionnement à rayonnement normal ;
- dans lequel le dispositif vestimentaire, dans le mode de fonctionnement normal, est configuré pour fonctionner dans l'état de fonctionnement à rayonnement faible lorsqu'aucun état physiologique n'est prévu et qu'aucun paramètre vital ou environnemental exigeant une intervention immédiate n'est mesurée ; et dans lequel le dispositif vestimentaire est configuré pour commuter automatiquement en mode de fonctionnement à rayonnement normal si le logiciel d'évaluation a détecté la présence actuelle ou future d'un état physiologique problématique, en particulier un risque supérieur de syndrome de mort subite du nourrisson et/ou un sentiment de faim, ou la présence d'un paramètre vital ou environnemental dans une plage de valeurs critiques pour la santé.

13. Dispositif vestimentaire selon l'une quelconque des revendications précédentes,
- dans lequel le dispositif vestimentaire comprend un ou plusieurs capteurs de paramètres environnementaux choisis dans un groupe incluant :
• un thermomètre qui mesure la température ambiante ;
• un dispositif de mesure destiné à mesurer l'humidité de l'air ambiant ;
• des gaz, en particulier CO₂ ;
• un capteur UV destiné à détecter une dose de rayonnement UV cumulative, en particulier une dose de rayonnement UV cumulative quotidienne ;
- et/ou dans lequel les capteurs du dispositif vestimentaire destiné à détecter les paramètres vitaux comprennent des capteurs supplémentaires choisis dans un groupe comprenant :
• un capteur d'accélération destiné à détecter la position de l'enfant (position couchée sur le dos, position couchée sur le ventre) ;
• un capteur de température destiné à détecter la température cutanée ;
• un microphone destiné à détecter les bruits ambiants et/ou les bruits émis par l'enfant ;
• une caméra vidéo, en particulier une caméra d'imagerie thermique.

14. Dispositif vestimentaire selon l'une quelconque des revendications précédentes, dans lequel le logiciel de prévision comprend au moins un modèle prédictif destiné à prévoir l'au moins un état physiologique, dans lequel l'au moins un modèle prédictif est un modèle généré par une méthode d'apprentissage machine sur la base d'un jeu de données d'entraînement.

15. Système comprenant le dispositif vestimentaire selon l'une quelconque des revendications précédentes et un ou plusieurs des constituants supplémentaires suivants :
- le dispositif de télécommunication portatif, dans lequel un logiciel utilisateur est installé sur le dispositif de télécommunication portatif, dans lequel le logiciel utilisateur est interfonctionnel avec le logiciel d'évaluation et est configuré pour afficher les résultats de prévision reçus du dispositif portatif par l'intermédiaire de l'interface à l'utilisateur et/ou pour permettre à l'utilisateur de configurer le logiciel d'évaluation ; et/ou
- le système serveur-ordinateur ; et/ou
- une station de base à laquelle un ou plusieurs capteurs externes sont couplés pour mesurer les paramètres vitaux de l'enfant ou des paramètres environnementaux des environs de l'enfant ; et/ou
- un ou plusieurs des capteurs externes, en particulier une caméra vidéo, en particulier une caméra vidéo d'imagerie thermique.

16. Procédé de fourniture d'un dispositif vestimentaire (200) destiné à surveiller l'état physiologique d'un enfant (300), comprenant les étapes consistant à :
- fournir (102) un jeu de données d'entraînement comprenant une pluralité de jeux de données, dans lequel dans chaque jeu de données au moins un état physiologique de l'enfant est stocké en lien avec des paramètres vitaux de l'enfant, dans lequel les paramètres vitaux comprennent au moins le rythme cardiaque, la saturation en oxygène et la fréquence respiratoire ;
- réaliser (104) un processus d'apprentissage machine sur les données d'entraînement afin de générer au moins un modèle prédictif (520, 620), dans lequel le modèle est configuré pour prévoir l'état physiologique de l'enfant sur la base d'au moins le rythme cardiaque, la saturation en oxygène et la fréquence respiratoire ;
- installer (106) un logiciel d'évaluation (408) incluant l'au moins un modèle prédictif (410, 520, 620) sur le dispositif vestimentaire, dans lequel le dispositif est conçu pour être porté sur le corps d'un enfant (300), dans lequel l'enfant est un bébé ou un tout-petit, dans lequel le dispositif comprend :
• un ou plusieurs capteurs (412, 414, 416) destinés à détecter une pluralité de paramètres vitaux de l'enfant, dans lequel les paramètres vitaux comprennent au moins le rythme cardiaque, la saturation en oxygène et la fréquence respiratoire, dans lequel le logiciel d'évaluation est configuré pour utiliser l'au moins un modèle prédictif pour prévoir l'état physiologique sur la base du rythme cardiaque, de la saturation en oxygène et de la fréquence respiratoire détectés par les capteurs, dans lequel au moins un parmi les capteurs de paramètres vitaux est configuré pour mesurer la glycémie sanguine de l'enfant de manière non invasive ; et
• une interface (403, 404) destinée à transmettre un résultat de prévision lié à l'au moins un état physiologique à un dispositif de télécommunication mobile (302) d'un utilisateur et/ou à un système serveur-ordinateur, dans lequel l'au moins un état physiologique est un état de risque supérieur de syndrome de mort subite du nourrisson, dans lequel le logiciel d'évaluation est configuré pour utiliser au moins le rythme cardiaque, la saturation en oxygène et la fréquence respiratoire en entrée pour prévoir la présence d'un risque supérieur de syndrome de mort subite du nourrisson,
dans lequel le logiciel d'évaluation est configuré pour prévoir un état physiologique supplémentaire sous la forme d'un sentiment de faim actuel ou futur chez l'enfant en fonction d'au moins la glycémie sanguine mesurée, et/ou pour prévoir un temps d'apparition futur du sentiment de faim, dans lequel le sentiment de faim est prévu lorsque la glycémie sanguine actuelle ou future est inférieure à une valeur limite prédéfinie,
• dans lequel le logiciel d'évaluation est couplé en communication à un appareil de préparation de nourriture électronique (702) directement ou au moyen d'une application logicielle du dispositif de télécommunication, dans lequel le logiciel d'évaluation et éventuellement également l'application logicielle du dispositif de télécommunications sont configurés pour amener l'appareil électronique à préparer de la nourriture pour l'enfant en réponse à la prévision selon laquelle l'enfant éprouve de la faim maintenant ou l'éprouvera à l'avenir.
